(19) Europäisches Patentamt
European Patent Office
Office européen des brevets

(11) EP 2 447 376 A1

(12) EUROPEAN PATENT APPLICATION

(43) Date of publication:
**02.05.2012 Bulletin 2012/18**

(51) Int Cl.:
***C12Q 1/68*** *(2006.01)*

(21) Application number: **11187128.1**

(22) Date of filing: **28.10.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.10.2010 JP 2010242836**
**27.10.2011 JP 2011235652**

(71) Applicant: **Arkray, Inc.**
**Kyoto-shi, Kyoto 601-8045 (JP)**

(72) Inventor: **Kurose, Kaoru**
**Kyoto-shi, Kyoto 601-8045 (JP)**

(74) Representative: **Jones, Elizabeth Louise**
**Dehns**
**St Bride's House**
**10 Salisbury Square**
**London**
**EC4Y 8JD (GB)**

# (54) Polymorphism detection probe, polymorphism detection method, evaluation of drug efficacy, and polymorphism detection kit

(57) The invention provides a probe which detects a polymorphism in the MDR1 gene. The probe has a P1 and/or a P2 oligonucleotide. The P1 oligonucleotide has a sequence that is complementary to a first base sequence, in which the first base sequence is a partial sequence of SEQ ID NO: 2 having a length of from 13 bases to 68 bases and including the 288th to 300th bases of SEQ ID NO: 2. The base complementary to the 288th base is labeled with a fluorescent dye. The P2 oligonucleotide has a sequence that is complementary to a second base sequence, in which the second base sequence is a partial sequence of SEQ ID NO: 2 having a length of from 6 bases to 93 bases and including the 300th to 305th bases of SEQ ID NO: 2. The base complementary to the 305th base is labeled with a fluorescent dye.

EP 2 447 376 A1

## Description

BACKGROUND

Field of the Invention

The present invention relates to a probe for detecting polymorphism, a method of detecting polymorphism, an assessment or evaluation of drug efficacy, and a kit for detecting polymorphism.

Related Art

A multidrug resistance gene MDR1, that may be also referred to as ATP-binding Cassette Sub-family B Member 1(ABCB1), encodes a transporter of various drugs, and defines in vivo pharmacokinetics of various drugs including digoxin.
It is regarded that the C3435T mutation, which is a mutation of the substitution of a cytosine (C) at 3435th position in the exon 26 of the MDR1 gene with a thymidine (T), changes the expression amount of P-glycoprotein derived from the MDR1 gene, whereby the in vivo pharmacokinetics of various drugs changes (for example, Proc. Natl. Acad. Sci. USA., 2000, vol. 97, no. 7, pp. 3473-3478).
It is also known that the expression amount of P-glycoprotein derived from the MDR1 gene is affected by the G2677A/T mutation, which is a mutation of the substitution of a guanine (G) at 2677th position in the exon 21 of the MDR1 gene with an adenine (A) or thymidine (T). It is further known that the C3435T mutation and the G2677A/T mutation simultaneously occur at a high rate.
Polymerase chain reaction-restriction fragment length polymorphism (PCR-RFLP) has been known as a method of detecting the C3435T mutation. In the PCR-RFLP, PCR is carried out using primers which have been designed so as to amplify a region containing the 3435th base in the exon 26 of the MDR1 gene. The products obtained by the amplification are subjected to cleaving with a restriction enzyme, which is selected so that the presence or absence of a cleaving thereby depends on whether the mutation of the 3435th base exists or not. The resultant is then electrophoresed to detect whether the products obtained by the amplification have been cleaved or not (for example, Genet. Mol. Res., 2010, 9(1), pp. 34-40).
As a method of detecting the C3435 mutation and the G2677A/T mutation, a method in which the C3435 mutation is detected by PCR-RFLP and the G2677A/T mutation is detected by a sequence analysis method has been known (for example, Br. J. Clin. Pharmcol., 2005, 59(3), pp. 365-370).
On the other hand, a method in which a region containing a mutation is amplified by PCR; then, melting curve analysis is carried out using a nucleic acid probe that has been labeled with a fluorescent dye; and, based on the results of the melting curve analysis, the mutation in the base sequence is analyzed has been known (for example, Clin. Chem., 2000, 46;5, pp. 631-635; and Japanese Patent Application Laid-Open (JP-A) No. 2002-119291).
A method of detecting the C3435T mutation in the exon 26 of the MDR1 gene by using the similar nucleic acid probe as described above has been known (for example, Japanese Patent Publication No. 4454366).

SUMMARY

The PCR-RFLP described in Genet. Mol. Res., 2010,9(1), pp. 34-40 and Br. J. Clin. Pharmcol., 2005, 59(3), pp. 365-370 needs to carry out PCR and then collect the amplification products, and treat them with a restriction enzyme. Therefore, there may be a risk that the amplification products may contaminate the following reaction system, which may result in a false-positive or false-negative. Further, since the restriction enzyme treatment is carried out after the completion of PCR and then the resultant is electrophoresed, it may take a very long time until the detection. In addition, this method may be hard to be automated due to complexed operations thereof.
The technique described in Japanese Patent Publication No. 4454366 is capable of detecting a C3435T mutation in the exon 26 of the MDR1 gene, but was not able to detect a G2677A/T mutation in the exon 21 of the MDR1 gene.
The present invention provides a probe for detecting a polymorphism which may enable to detect the G2677A/T mutation in the exon 21 of the MDR1 gene easily with a high sensitivity, a method of detecting a polymorphism by using the probe, a method of evaluating the efficacy of a drug by using the probe, and a kit for detecting a polymorphism by using the probe.
One exemplary embodiment of a first aspect of the present invention is <1> a probe which detects a polymorphism in the MDR1 gene, the probe comprising at least one fluorescently labeled oligonucleotide selected from the group consisting of a P1 oligonucleotide and a P2 oligonucleotide,
the P1 oligonucleotide having (1) a sequence that is complementary to a first base sequence or (2) a sequence that is homologous to (1), the first base sequence being a partial sequence of SEQ ID NO:2 having a length of from 13 bases to 68 bases and comprising the 288th to 300th bases of SEQ ID NO:2, and the P1 oligonucleotide having, as a base complementary to the 288th base, a base that is labeled with a first fluorescent dye, and
the P2 oligonucleotide having (3) a sequence that is complementary to a second base sequence or (4) a sequence that is homologous to (3), the second base sequence being a partial sequence of SEQ ID NO:2 having a length of from 6 bases to 93 bases and comprising the 300th to 305th bases of SEQ ID NO:2, and the P2 oligonucleotide having, as a base complementary to the 305th base, a base that is labeled with a second fluorescent dye.

Another exemplary embodiment of the first aspect of the present invention is <2> the probe of <1>, wherein the base labeled with the first fluorescent dye is at a position of any one of 1st to 3rd positions from the 3' end of the P1 oligonucleotide, and the base labeled with the second fluorescent dye is at a position of any one of 1 st to 3rd positions from the 5' end of the P2 oligonucleotide.

Another exemplary embodiment of the first aspect of the present invention is <3> the probe of <1> or <2>, wherein the base labeled with the first fluorescent dye is at the 3' end of the P1 oligonucleotide, and the base labeled with the second fluorescent dye is at the 5' end of the P2 oligonucleotide.

Another exemplary embodiment of the first aspect of the present invention is <4> the probe of any one of <1> to <3>, wherein the fluorescence intensity of the fluorescently labeled oligonucleotide when hybridized to its target sequence is larger or smaller than the fluorescence intensity when not hybridized to its target sequence.

Another exemplary embodiment of the first aspect of the present invention is <5> the probe of any one of <1> to <4>, wherein the fluorescence intensity of the fluorescently labeled oligonucleotide when hybridized to its target sequence is smaller than the fluorescence intensity when not hybridized to its target sequence.

Another exemplary embodiment of the first aspect of the present invention is <6> the probe of any one of <1 > to <5>, wherein the length of the P1 oligonucleotide is in a range of from 13 bases to 56 bases and the length of the P2 oligonucleotide is in a range of from 6 bases to 29 bases.

Another exemplary embodiment of the first aspect of the present invention is <7> the probe of any one of <1> to <6>, wherein the length of the P1 oligonucleotide is in a range of from 13 bases to 26 bases and the length of the P2 oligonucleotide is in a range of from 6 bases to 23 bases.

Another exemplary embodiment of the first aspect of the present invention is <8> the probe of any one of <1> to <7>, wherein the length of the P1 oligonucleotide is in a range of from 13 bases to 21 bases and the length of the P2 oligonucleotide is in a range of from 6 bases to 18 bases.

Another exemplary embodiment of the first aspect of the present invention is <9> the probe of any one of <1> to <8>, being a probe for melting curve analysis.

Another exemplary embodiment of the first aspect of the present invention is <10> the probe of any one of <1> to <9>, comprising at least two fluorescently labeled oligonucleotides that are different from each other in terms of bases complementary to the 300th base of the base sequence of SEQ ID NO:2 and have a C, a T or an A as the complementary bases.

One exemplary embodiment of a second aspect of the present invention is <11> a method of detecting a polymorphism of the MDR1 gene, the method comprising using the probe of any one of <1> to <10>.

Another exemplary embodiment of the second aspect of the present invention is <12> the method of <11>, comprising:

(I) obtaining a hybrid formed of a single-stranded nucleic acid and the probe by hybridizing the fluorescently labeled oligonucleotide and the single-stranded nucleic acid by contacting the single-stranded nucleic acid in a sample with the probe;
(II) measuring a change of a signal based on dissociation of the hybrid by changing the temperature of the sample comprising the hybrid in order to dissociate the hybrid;
(III) determining, as a melting temperature, a temperature at which the hybrid dissociates based on the signal variation; and
(IV) checking for presence of the polymorphism of the MDR1 gene based on the melting temperature.

Another exemplary embodiment of the second aspect of the present invention is <13> the method of <11> or <12>, further comprising obtaining the single-stranded nucleic acid by performing amplification of a nucleic acid before the (I) obtaining of a hybrid or during the (I) obtaining of a hybrid.

Another exemplary embodiment of the second aspect of the present invention is <14> the method of any one of <11> to <13>, further comprising contacting a probe with the single-stranded nucleic acid in the sample, the probe being capable of detecting a mutation of the 256th base of the base sequence of SEQ ID NO:1.

Another exemplary embodiment of the second aspect of the present invention is <15> the method of <14>, wherein the probe that is capable of detecting a mutation of the 256th base of the base sequence of SEQ ID NO:1 is a fluorescently labeled oligonucleotide having a base sequence that is complementary to a sequence having a length of from 9 bases to 50 bases that starts from the 248th base of the base sequence of SEQ ID NO:1.

One exemplary embodiment of a third aspect of the present invention is <16> a method of evaluating a drug, comprising:

detecting a polymorphism in the MDR1 gene by the method of any one of <11> to <15>; and
evaluating a resistance of a source of the sample to the drug or an effect of the drug based on a result of the detection.

One exemplary embodiment of a fourth aspect of the present invention is <17> a kit for detecting a polymorphism, comprising the probe of any one of <1 > to < 10>.

Another exemplary embodiment of the fourth aspect of the present invention is <18> the kit of <17>, further comprising a primer that is capable of performing amplification by using, as a template, a region that is in the base sequence of SEQ ID NO:2 and comprises a sequence to which the P1 oligonucleotide or the P2 oligonucleotide hybridizes.

Another exemplary embodiment of the fourth aspect of the present invention is <19> the kit of <17> or <18>, further comprising a fluorescently labeled oligonucleotide having a base sequence that is complementary to a sequence having a length of from 9 bases to 50 bases that starts from the 248th base of the base sequence of SEQ ID NO: 1.

BRIEF DESCRIPTION OF THE DRAWINGS

Fig. 1A is an example of a melting curve of a nucleic acid mixture.
Fig. 1B is an example of a differential melting curve of a nucleic acid mixture.
Fig. 2A is an example of a differential melting curve of an exemplary embodiment of the polymorphism detection probe and a nucleic acid which is non-complementary to the probe.
Fig. 2B is an example of a differential melting curve of an exemplary embodiment of the polymorphism detection probe and a nucleic acid which is complementary to the probe.
Fig. 3A is an example of a differential melting curve of an exemplary embodiment of the polymorphism detection probe and a nucleic acid which is non-complementary to the probe.
Fig. 3B is an example of a differential melting curve of an exemplary embodiment of the polymorphism detection probe and a nucleic acid which is non-complementary to the probe.
Fig. 4A is an example of a differential melting curve of an exemplary embodiment of the polymorphism detection probe and a nucleic acid which is complementary to the probe.
Fig. 4B is an example of a differential melting curve of an exemplary embodiment of the polymorphism detection probe and a nucleic acid which is non-complementary to the probe.
Fig. 5A is an example of a differential melting curve of an exemplary embodiment of the polymorphism detection probe and a nucleic acid which is non-complementary to the probe.
Fig. 5B is an example of a differential melting curve of an exemplary embodiment of the polymorphism detection probe and a mixture of a nucleic acid which is non-complementary to the probe and a nucleic acid which is complementary to the probe.
Fig. 6A is an example of a differential melting curve of an exemplary embodiment of the polymorphism detection probe and a mixture of a nucleic acid which is non-complementary to the probe and a nucleic acid which is complementary to the probe.
Fig. 6B is an example of a differential melting curve of an exemplary embodiment of the polymorphism detection probe and a mixture of a nucleic acid which is non-complementary to the probe and a nucleic acid which is complementary to the probe.
Fig. 7A is an example of a differential melting curve of an exemplary embodiment of the polymorphism detection probe and a mixture of a nucleic acid which is non-complementary to the probe and a nucleic acid which is complementary to the probe.
Fig. 7B is an example of a differential melting curve of an exemplary embodiment of the polymorphism detection probe and a nucleic acid which is non-complementary to the probe.
Fig. 8 is an example of a differential melting curve of an exemplary embodiment of the polymorphism detection probe and a nucleic acid which is non-complementary to the probe.
Fig. 9 is an example of a differential melting curve of an exemplary embodiment of the polymorphism detection probe and a nucleic acid which is complementary to the probe.
Fig. 10 is an example of a differential melting curve of an exemplary embodiment of the polymorphism detection probe and a mixture of a nucleic acid which is non-complementary to the probe and a nucleic acid which is complementary to the probe.

DETAILED DESCRIPTION

Polymorphism detection probe

The probe of one exemplary embodiment of one aspect of the invention, that may be simply referred to as "the probe" hereinafter, is a probe having at least one fluorescently labeled oligonucleotide selected from the group consisting of a P1 oligonucleotide and a P2 oligonucleotide, that may be simply referred to as "the specific fluorescently labeled oligonucleotide(s)" hereinafter.

The P1 oligonucleotide has (1) a sequence that is complementary to a first base sequence or (2) a sequence that is homologous to (1), in which the first base sequence is a partial sequence of SEQ ID NO: 2 having a length of from 13 bases to 68 bases and including the 288th to 300th bases of SEQ ID NO: 2. The P1 oligonucleotide has, as a base complementary to the 288th base, a base which is labeled with a fluorescent dye.

The P2 oligonucleotide has (3) a sequence that is complementary to a second base sequence or (4) a sequence that is homologous to (3), in which the second base sequence is a partial sequence of SEQ ID NO: 2 having a length of from 6 bases to 93 bases and including the 300th to 305th bases of SEQ ID NO: 2. The P2 oligonucleotide has, as a base complementary to the 305th base, a base which is labeled with a fluorescent dye.

The probe may enable to detect the G2677A/T mutation in the exon 21 of the MDR1 gene with a high sensitivity and easily by containing at least one of the specific fluorescently labeled oligonucleotides.

The base sequence of SEQ ID NO: 2 is a partial base sequence of the exon 21 of the MDR1 gene, and the 2677th base in the exon 21 of the MDR1 gene corresponds to the 300th base of the base sequence of SEQ ID NO: 2. The 300th base of the base sequence of SEQ ID NO: 2 is a G (guanine) in a wild type, while it mutates into an A (adenine) or a T (thymine) in a mutant type.

In the P1 oligonucleotide, "a base that is complimentary to the 288th base" is a cytosine (C) that is complementary to a guanine (G) of the 288th base of the base sequence of SEQ ID NO: 2.

An expression of "a sequence that is homologous to one base sequence" herein means a sequence having a similarity to the one base sequence of 80% or more, preferably 90% or more, and further preferably 95% or more.

In the P1 oligonucleotide, this fluorescently labeled complementary base C preferably exists at a position of any one of 1 st to 3rd positions from its 3' end, and more preferably at its 3' end.

The P1 oligonucleotide is a probe that is capable of detecting a polymorphism of the 300th base of the base sequence of SEQ ID NO: 2. This 300th base of the base sequence of SEQ ID NO: 2 corresponds to the 2677th base in the exon 21 of the MDR1 gene, and is a G in a wild type and a T or an A in a mutant type. Therefore, a base in the P1 oligonucleotide which is complimentary to this base may be preferably a C, an A or a T.

In other words, it may be preferable that the P1 oligonucleotide includes at least one selected from the group consisting of (i) an oligonucleotide having a sequence complementary to a sequence of a wild type, (ii) an oligonucleotide having a sequence which is homologous to (i), (iii) an oligonucleotide having a sequence complementary to a sequence of a mutant type, and (iv) an oligonucleotide having a sequence which is homologous to (iii).

The length of the P1 oligonucleotide is in a range of from 13 bases to 68 bases, and may be preferably in a range of from 13 bases to 56 bases, more preferably from 13 bases to 26 bases, and further preferably from 13 bases to 21 bases. When the length is within any one of these ranges, for example, the sensitivity for detecting a polymorphism may be further improved.

The melting temperature (Tm), that is a temperature at which a hybrid formed of the P1 oligonucleotide and its complementary sequence dissociates, may be adjusted to a desired value by varying the length of the P1 oligonucleotide.

Examples of a base sequence of the P1 oligonucleotide are shown in Table 1 below, but the invention is not limited to these.

Table 1 further shows the Tms of hybrids formed of various fluorescently labeled oligonucleotides and oligonucleotides complimentary to target sequences in which the base corresponding to the 300th base of SEQ ID NO: 2 is a G, a T or an A. The Tms were calculated by using MeltCalc© 99 FREE (http://www.meltcalc.com/) and under the conditions of: Oligoconc. [μM] of 0.2 and Na eq. [mM] of 50.

Table 1

| SEQ ID NO. | BASE SEQUENCE | Length | Tm (°C) | | | Δ (G-T) | Δ (G-A) |
|---|---|---|---|---|---|---|---|
| | | | G | T | A | | |
| SEQ ID NO. 11 | Cacottctagttc | 13 | 33.5 | 25.1 | 24.2 | 8.4 | 9.3 |
| SEQ ID NO. 8 | cttcccagCaccttctagttc | 21 | 54.0 | 44.5 | 44.5 | 9.5 | 9.5 |
| SEQ ID NO. 12 | caccttcccagCaccttctagttc | 24 | 58.5 | 50.8 | 50.8 | 7.7 | 7.7 |
| SEQ ID NO. 13 | tcaccttcccagCaccttctagttc | 25 | 59.5 | 52.1 | 52.1 | 7.4 | 7.4 |
| SEQ ID NO. 14 | ctcaccttcccagCaccttctagttc | 26 | 60.1 | 53.1 | 53.1 | 7.0 | 7.0 |
| SEQ ID NO. 15 | tttgactcaccttcccagCaccttctagttc | 31 | 62.8 | 57.1 | 57.2 | 5.7 | 5.6 |
| SEQ ID NO. 16 | tttagtttgactcaccttcccagCaccttctagttc | 36 | 63.6 | 58.9 | 58.9 | 4.7 | 4.7 |
| SEQ ID NO. 17 | tcatatttagtttgactcaccttcccagCaccttctagttc | 41 | 64.1 | 60.4 | 59.9 | 3.7 | 4.2 |
| SEQ ID NO. 18 | atcaatcatatttagtttgactcaccttcccagCaccttctagttc | 46 | 64.8 | 61.2 | 61.1 | 3.6 | 3.7 |
| SEQ ID NO. 19 | aattaatcaatcatatttagtttgactcaccttcccagCaccttctagttc | 51 | 64.5 | 61.2 | 61.2 | 3.3 | 3.3 |
| SEQ ID NO. 20 | tacttaattaatcaatcatatttagtttgactcaccttcccagCaccttctagttc | 56 | 64.6 | 61.6 | 61.6 | 3.0 | 3.0 |
| SEQ ID NO. 21 | tactctacttaattaatcaatcatatttagtttgactcaccttcccagCaccttctagttc | 61 | 65.3 | 62.7 | 62.6 | 2.6 | 2.7 |
| SEQ ID NO. 22 | tactttactctacttaattaatcaatcatatttagtttgactcaccttcccagCaccttctagttc | 66 | 65.6 | 63.2 | 63.1 | 2.4 | 2.5 |
| SEQ ID NO. 23 | atactttactctacttaattaatcaatcatatttagtttgactcaccttcccagCaccttctagttc | 67 | 65.5 | 63.1 | 63.1 | 2.4 | 2.4 |
| SEQ ID NO. 24 | aatactttactctacttaattaatcaatcatatttagtttgactcaccttcccagCaccttctagttc | 68 | 65.6 | 63.2 | 63.2 | 2.4 | 2.4 |
| SEQ ID NO. 42 | gaatactttactctacttaattaatcaatcatatttagtttgactcaccttcccagCaccttctagttc | 69 | 65.7 | 63.3 | 65.3 | 2.4 | 0.4 |

The oligonucleotides exemplified in Table 1 are limited to those complementary to sequences in which the 300th base of SEQ ID NO: 2 is a guanine (G) (namely, a wild type). Oligonucleotides which are the base complementary to sequences in which the 300th base of SEQ ID NO: 2 is a thymine (T) or an adenine (A), i.e., oligonucleotides in which the cytosine (C) in Table 1 is replaced by an A or a T, may be also exemplified in the similar manner.

The difference between a Tm exhibited in the case where the P1 oligonucleotide is hybridized with a DNA having its completely complementary base sequence and a Tm exhibited in the case where the P1 oligonucleotide is hybridized with a DNA having its complementary base sequence except a base which corresponds to the 300th base of SEQ ID NO: 2 and is non-complementary to the P1 oligonucleotide may be preferably 3.0°C or more, more preferably 7.0°C or more, and further preferably 9.0°C or more. When the difference of the Tms is 3.0°C or more, for example, a mutation of the 300th base of SEQ ID NO: 2 may be detected with a higher sensitivity.

In the P2 oligonucleotide, "a base that is complimentary to the 305th base" is a C (cytosine) that is complementary to a guanine (G) of the 305th base of the base sequence of SEQ ID NO: 2.

In the P2 oligonucleotide, this fluorescently labeled complementary base C preferably exists at a position of any one of 1 st to 3rd positions from its 5' end, and more preferably at its 5' end. The sensitivity for detecting a polymorphism may be further improved thereby. In addition, the fluorescently labeled P2 oligonucleotide may be obtained with a good productivity.

The P2 oligonucleotide is a probe that is capable of detecting a polymorphism of the 300th base of the base sequence of SEQ ID NO: 2. This 300th base of the base sequence of SEQ ID NO: 2 corresponds to the 2677th base in the exon 21 of the MDR1 gene, and is a G in a wild type and a T or an A in a mutant type. Therefore, a base in the P2 oligonucleotide which is complimentary to this base may be preferably a C, an A or a T.

In other words, it may be preferable that the P2 oligonucleotide includes at least one selected from the group consisting of (i) an oligonucleotide having a sequence which is complementary to a sequence of a wild type, (ii) an oligonucleotide having a sequence which is homologous to (i), (iii) an oligonucleotide having a sequence which is complementary to a sequence of a mutant type, and (iv) an oligonucleotide having a sequence which is homologous to (iii).

The length of the P2 oligonucleotide is in a range of from 6 bases to 93 bases, and may be preferably in a range of from 6 bases to 29 bases, more preferably from 6 bases to 29 bases, and further preferably from 6 bases to 18 bases. When the length is within any one of these ranges, for example, the sensitivity for detecting a polymorphism may be further improved.

The melting temperature (Tm), that is a temperature at which a hybrid formed of the P2 oligonucleotide and its complementary sequence dissociates, may be adjusted to a desired value by varying the length of the P2 oligonucleotide.

Examples of a base sequence of the P2 oligonucleotide are shown in Table 2 below, but the invention is not limited to these.

Table 2 further shows the Tms of hybrids formed of various fluorescently labeled oligonucleotides and oligonucleotides complimentary to target sequences in which the base corresponding to the 300th base of SEQ ID NO: 2 is a T, a G or an A. The Tms were calculated in the same manner as described above.

Table 2

| SEQ ID NO. | BASE SEQUENCE | Length | Tm (°C) | | | Δ(T-G) | Δ(T-A) |
|---|---|---|---|---|---|---|---|
| | | | T | G | A | | |
| SEQ ID NO. 25 | cccagA | 6 | -7.3 3 | -19. 7 | -22.6 | 12.4 | 15.3 |
| SEQ ID NO. 9 | cccagAaccttctagttc | 18 | 47.6 | 41.6 | 40.5 | 6.0 | 7.1 |
| SEQ ID NO. 26 | cccagAaccttctagttctttct | 23 | 53. 2 | 49. 2 | 48.3 | 4.0 | 4. 9 |
| SEQ ID NO. 27 | cccagAaccttctagttctttcttatct | 28 | 55.6 | 50.9 | 51.1 | 4. 7 | 4.5 |
| SEQ ID NO. 28 | cccagAaccttctagttctttcttatctt | 29 | 56.0 | 53. 1 | 52.3 | 2. 9 | 3.7 |
| SEQ ID NO. 29 | cccagAaccttctagttctttcttatctttcag | 33 | 58.5 | 56. 1 | 55.5 | 2.4 | 3.0 |
| SEQ ID NO. 30 | cccagAaccttctagttctttcttatctttcagtgctt | 38 | 62.2 | 60.4 | 59.8 | 1.8 | 2.4 |
| SEQ ID NO. 31 | cccagAaccttctagttctttcttatctttcagtgcttgtcca | 43 | 65.1 | 63.7 7 | 63.2 | 1.4 | 1.9 |
| SEQ ID NO. 32 | cccagAaccttctagttctttcttatctttcagtgcttgtccagacaa | 48 | 66.5 | 65. 3 | 64.9 | 1.2 | 1.6 |
| SEQ ID NO. 33 | cccagAaccttctagttatttcttatctttcagtgcttgtccagacaacattt | 53 | 67.1 1 | 66.1 | 65.6 | 1.0 | 1.5 |
| SEQ ID NO. 34 | cccagAaccttctagttctttcttatctttcagtgcttgtccagacaacattttcatt | 58 | 67.5 | 66. 5 | 66.1 | 1.0 | 1.4 |
| SEQ ID NO. 35 | cccagAaccttctagttctttcttatctttcagtgcttgtccagacaacattttcatttcaac | 63 | 68.0 | 67. 2 | 66.8 | 0.8 | 1.2 |
| SEQ ID NO. 36 | cccagAaccttctagttctttcttatctttcagtgcttgtccagacaacattttcatttcaacaactc | 68 | 68.8 | 68. 1 | 67.7 | 0.7 | 1.1 |
| SEQ ID NO. 37 | cccagAaccttctagttctttcttatctttcagtgcttgtccagacaacattttcatttcaacaactcctgct | 73 | 70.5 | 69. 9 | 69. 6 | 0.6 | 0.9 |
| SEQ ID NO. 38 | cccagAaccttctagttctttcttatctttcagtgcttgtccagacaacattttcatttcaacaactcctgctattgc | 78 | 70. 7 | 70.1 | 68.9 | 0.6 | 1.8 |
| SEQ ID NO. 39 | cccagAaccttctagttctttcttatctttcagtgcttgtccagacaacattttcatttcaacaactcctgctattgcaatga | 83 | 71.3 | 70.5 | 70.3 | 0.8 | 1.0 |
| SEQ ID NO. 40 | cccagAaccttctagttctttcttatctttcagtgcttgtccagacaacattttcatttcaacaactcctgctattgcaatgatgggt | 88 | 72.1 | 71.6 | 71.3 | 0.5 | 0.8 |
| SEQ ID NO. 43 | cccagAaccttctagttctttcttatctttcagtgcttgtccagacaacattttcatttcaacaactcctgctattgcaatgatgggtacaat | 93 | 71.9 | 71.4 | 71.2 | 0.5 | 0.7 |
| SEQ ID NO. 43 | cccagAaccttctagttctttcttatctttcagtgcttgtccagacaacattttcatttcaacaactcctgctattgcaatgatgggtacaatt | 94 | 71.8 | 71.4 | 71.1 | 0.4 | 0.7 |

The oligonucleotides exemplified in Table 2 are limited to those complementary to sequences in which the 300th base of SEQ ID NO: 2 is a thymine (T) (namely, a mutant type). Oligonucleotides which are the base complementary to sequences in which the 300th base of SEQ ID NO: 2 is a guanine (G) or an adenine (A), i.e., oligonucleotides in which the in Table 2 is replaced by a C or a T, may be also exemplified in the similar manner.

The difference between a Tm exhibited in the case where the P2 oligonucleotide is hybridized with a DNA having its completely complementary base sequence and a Tm exhibited in the case where the P2 oligonucleotide is hybridized with a DNA having its complementary base sequence except a base which corresponds to the 300th base of SEQ ID NO: 2 and is non-complementary to the P2 oligonucleotide may be preferably 2.8°C or more, more preferably 4.0°C or more, and further preferably 6.0°C or more. When the difference of the Tms is 2.8°C or more, for example, a mutation of the 300th base of SEQ ID NO: 2 can be detected with a higher sensitivity.

The fluorescently labeled oligonucleotide, i.e., the probe, may be preferably a fluorescently labeled oligonucleotide whose fluorescence intensity when hybridizing to its target sequence is decreased (quenched) or increased as compared to the fluorescence intensity when the fluorescently labeled oligonucleotide is not hybridizing to its target sequence. In particular, a fluorescently labeled oligonucleotide whose the fluorescence intensity when the fluorescently labeled oligonucleotide is hybridizing to its target sequence is decreased as compared to the fluorescence intensity when the fluorescently labeled oligonucleotide is not hybridizing to its target sequence may be more preferable. A probe that uses the "fluorescence quenching phenomenon" is generally referred to as a guanine quenching probe, and it is known as Q PROBE®. In embodiments, the fluorescently labeled oligonucleotide may be more preferably designed so that its 3' or 5' end is a C and that is labeled with a fluorescent dye so that the fluorescence emission is reduced when the C at the 3' or 5' end comes close to a G.

The fluorescent dye is not particularly limited. Examples of the fluorescent dye include fluorescein, phosphor, rhodamine and polymethine dye derivatives. Examples of commercially available products of such fluorescent dyes include, PACIFIC BLUE and BODIPY FL (trademarks, manufactured by Molecular Probes Inc.), FLUOREPRIME (trade name, manufactured by Amersham Pharmacia), FLUOREDITE (trade name, manufactured by Millipore Corporation), FAM (manufactured by ABI), Cy3 and Cy5 (manufactured by Amersham Pharmacia) and TAMRA (manufactured by Molecular Probes Inc.).

The detection conditions of the fluorescently labeled oligonucleotide are not particularly limited, and may be properly decided depending on the fluorescent dye to be used. For example, PACIFIC BLUE (described above) may be detected at a detection wavelength of from 445 nm to 480 nm; TAMRA (described above) may be detected at a detection wavelength of from 585 nm to 700 nm; and, BODIPY FL (described above) may be detected at a detection wavelength of from 520 nm to 555 nm. By using the probe having such fluorescent dye, the hybridization and the dissociation of the probe may be readily checked by the change of its fluorescence signal.

The fluorescently labeled oligonucleotide may have, for example, a phosphate group added to its 3' end. As described below, a target DNA, which is a DNA to be detected whether a polymorphism(s) exist(s) or not, may be prepared by a gene amplification method such as PCR. The fluorescently labeled oligonucleotide that has a phosphate group added to its 3' end may be made to coexist in a reaction solution of the amplification reaction by using the oligonucleotide in the amplification.

An expansion of the probe itself by the gene amplification reaction may be sufficiently suppressed by adding a phosphate group to the 3' end of the fluorescently labeled oligonucleotide. In embodiments, the similar effect may also be obtained by adding such a labeling substance (fluorescent dye) to the 3' end.

Specific examples of the oligonucleotide which has the above-described base sequence and the C base at its 5' or 3' end is labeled with a fluorescent dye are shown below (the base described by a capital letter each represents a mutated site, and the P represents a phosphate group). However, the fluorescently labeled oligonucleotide is not limited to those described below.

Table 3

| | BASE SEQUENCE | Length | SEQ ID NO. |
|---|---|---|---|
| P1 | Caccttctagttc-(TAMRA) | 13 | SEQ ID NO. 11 |
| | cttcccagCaccttctagttc-(TAMRA) | 21 | SED ID NO. 8 |
| | caccttcccagCaccttctagttc-(TAMRA) | 24 | SEQ ID NO. 12 |
| | tcaccttcccagCaccttctagttc-(TAMRA) | 25 | SEQ ID NO. 13 |
| | ctcaccttcccagCaccttctagttc-(TAMRA) | 26 | SEQ ID NO. 14 |
| | tacttaattaatcaatcatatttagtttgactcaccttcccagCaccttctagttc-(TAMRA) | 56 | SEQ ID NO. 20 |

(continued)

| | BASE SEQUENCE | Length | SEQ ID NO. |
|---|---|---|---|
| P2 | (BODYPY FL)-cocagA-P | 6 | SEQ ID NO. 25 |
| | (BODYPY FL)-cccagAaccttctagttc-P | 18 | SEQ ID NO. 9 |
| | (BODYPY FL)-cccagAaccttctagttctttct-P | 23 | SEQ ID ND,26 |
| | (BODYPY FL)-cccagAaccttctagttctttcttatct-P | 28 | SEQ ID NO 27 |
| | (BODYPY FL)-cccagAaccttctagttctttcttatctt-P | 29 | SEQ ID NO. 28 |

The fluorescently labeled oligonucleotide may be used as a probe for detecting a polymorphism of the MDR1 gene, specifically a polymorphism in the exon 21 of the gene.
Whether the 2677th base in the exon 21 of the MDR1 gene is a G, an A or a T can be distinguished by using, as probes for detecting polymorphism, at least two fluorescently labeled oligonucleotides, which are different from each other in the bases complementary to the 300th base of SEQ ID NO: 2 by independently having a C, a T or an A as the bases.
The at least two fluorescently labeled oligonucleotides may preferably contain fluorescent dyes different from each other. The polymorphisms may be distinguished with a higher sensitivity and more easily thereby.
The method of detecting a polymorphism is not particularly limited as long as it is a method that uses hybridization of the fluorescently labeled nucleotide and the sequence to be detected. As an example of the method of detecting a polymorphism that uses the fluorescently labeled nucleotide, a method of detecting a polymorphism that utilizes Tm analysis is described below.

Polymorphism detection method

The method of detecting a polymorphism of one exemplary embodiment of one aspect of the invention is a method of detecting a polymorphism of the MDR1 gene and includes at least the following (I) to (IV). The configuration of the method and conditions of the method are not particularly limited by the explanation described below as long as the method includes the use of the polymorphism detection probe.

(I) Obtaining a hybrid formed of a single-stranded nucleic acid and the probe by hybridizing the fluorescently labeled oligonucleotide and the single-stranded nucleic acid by contacting the single-stranded nucleic acid in a sample with the probe.
(II) Measuring a change of a signal based on dissociation of the hybrid by changing the temperature of the sample comprising the hybrid in order to dissociate the hybrid.
(III) Determining, as a melting temperature, a temperature at which the hybrid dissociates based on the signal variation.
(IV) Checking for presence of the polymorphism of the MDR1 gene based on the melting temperature.

The determination of the Tm in the (III) includes not only determining a dissociation temperature of the hybrid, but also determining the size of the differential value of the fluorescence signal which varies depending on the temperature while the hybrid is melting. The abundance ratio of a base sequence (DNA) having a polymorphism can be assessed by the size of the differential value.
A method of determining the abundance ratio of a wilt type and a particular mutant type is hereinafter explained as one exemplary embodiment of a method of quantitatively determining the abundance ratio of a base sequence having a polymorphism. It should be noted that the invention is not limited thereby.
First, for example, plural nucleic acid mixtures are prepared that each have different abundance ratios of two types of nucleic acid, a wild-type nucleic acid Wt and a mutant nucleic acid Mt. Melting curves are obtained with a melting curve analysis instrument for each of the plural nucleic acid mixtures.
Fig. 1A illustrates a melting curve expressing the relationship for a single nucleic acid mixture of a detection signal, such as a degree of light absorption or fluorescence intensity, to temperature. Fig. 1B illustrates a melting curve (also called a differential melting curve) expressing the relationship of the differential values of the detection signal to temperature. The melting temperature $Tm_W$ of the nucleic acid Wt and the melting temperature $Tm_M$ of the mutant nucleic acid Mt are detected from the peaks of the differential melting curve. Temperature ranges are then set to contain $Tm_W$ and $Tm_M$, respectively.
As a temperature range $\Delta T_W$ containing $Tm_M$ a temperature range can be set, for example, with a lower limit at the temperature at which the differential value of the detection signal reaches a minimum between $Tm_W$ and $Tm_M$, and an upper limit at the temperature corresponding to the tail of the detection signal peak. As the temperature range $\Delta T_M$ containing $Tm_M$, a temperature range can be set, for example, with an upper limit at the temperature at which the differential value of the detection signal reaches a minimum between $Tm_w$ and $Tm_M$, and with a lower limit at a temperature corresponding to the tail of the detection signal peak.

The temperature range $\Delta T_W$ and the temperature range $\Delta T_M$ can be set so as to have the same width as each other (for example 10°C) or set to have different widths from each other (for example a temperature range $Tm_w$ of 10°C, and a temperature range $Tm_M$ of 7°C). The temperature range $\Delta T_W$ and the temperature range $\Delta T_M$ can be set with widths from minus X°C to plus X°C from the temperature range Tm or the temperature range Tw, respectively, (for example, 15°C or less, or preferably 10°C or less).

Then, for each of the temperature range $\Delta T_W$ and the temperature range $AT_M$, respectively, a surface area is derived of an area bounded by a straight line passing through a point corresponding to the lower limit and a point corresponding to the upper limit of the respective temperature range of the differential melting curve and bounded by the differential melting curve itself (the shaded regions in Fig. 1B). A specific example of a method that can be employed for deriving the surface area is set out below. Derivation can be made according to the following Equality (1), in which f (T) is a differential value of the detection signal at temperature T, and B (T) is a base value at temperature T.

$$\text{Surface Area } S = \{f(T_{s+1}) - B(T_{s+1})\} + \{f(T_{s+2}) - B(T_{s+2})\} \text{ and so on up to } \{f(T_{e-1}) - B(T_{e-1})\} \qquad \text{Equality (1)}$$

In Equality (1), $T_s$ is the lower limit value of each of the temperature ranges, and $T_e$ is the upper limit value thereof. The base value B (T) at each temperature T is a value derived according to the following Equality (2), and represents the background level included in the detection signal. Influence from background included in the detection signal is removed by subtracting this base value from the differential value of the detection signal.

$$B(T) = a \times (T-T_s) + f(T_s) \qquad \text{Equality (2)}$$

In Equality (2), $a = \{f(T_e)-f(T_s)\} / (T_e-T_s)$.

Nucleic acids in a sample may be single-stranded nucleic acids or double-stranded nucleic acids. In the case where the nucleic acids are double-stranded nucleic acids, it may be preferable to include, for example, heating the double-stranded nucleic acids in the sample to melt (dissociate) them into single-stranded nucleic acids before the hybridization with the fluorescently labeled oligonucleotide. The dissociation of a double-stranded nucleic acid into single-stranded nucleic acids may enable the hybridization with the fluorescently labeled oligonucleotide.

The nucleic acids contained in a sample may be, for example, nucleic acids originally contained in a biological sample. In embodiments, in view of improving the detection accuracy, the nucleic acids contained in a sample may be preferably an amplification product obtained by amplification by PCR which uses, as a template, a region containing a mutation site(s) of the MDR1 gene using a nucleic acid originally contained in a biological sample. The length of the amplification products is not particularly limited. For example, it may be in a range if from 50 bases to 1000 bases, and may be preferably 80 bases to 200 bases. In embodiments, the nucleic acids in a sample may be, for example, cDNAs that have been synthesized from RNAs derived from a biological sample (for example, total RNAs or mRNAs) by reverse transcription PCR (RT-PCR).

The sample to which the method of detecting a polymorphism is applied is not particularly limited, as long as it is a sample in which the MDR1 gene exists. Specific examples of the sample include a sample of hemocytes such as leukocyte cells and a sample of whole blood. The method for collecting a sample, the method for preparing a sample that contains nucleic acids and the like are not limited. Conventionally known methods may be used therefor.

The rate of the addition amount of the probe relative to the content of the nucleic acids in a sample (in a molar ratio) is not particularly limited. In embodiments, the ratio may be preferably 1 time or less, more preferably 0.1 times or less, relative to DNAs in a sample. By this, for example, the signal can be detected sufficiently.

The "content of nucleic acids in a sample" may be, for example, a total of the nucleic acids to be detected in which a polymorphism to be detected has been generated and nucleic acids not to be detected in which the polymorphism to be detected has not been generated; or a total of amplification products containing a sequence in which a polymorphism to be detected has been generated and thus is to be detected and amplification products containing a sequence in which the polymorphism to be detected has not been generated and thus is not to be detected. Although the rate of the content of the nucleic acids to be detected to the content of the nucleic acids in a sample is usually unclear, the ratio of the addition amount of the probe (in a molar ratio) may preferably, as a consequence, become 10 times or less, more preferably 5 times or less, and further preferably 3 times or less, relative to the content of the nucleic acids to be detected (or amplification products containing a sequence to be detected). Although the lower limit of the rate is not particularly limited, it may be, for example, 0.001 times or more, preferably 0.01 times or more, more preferably 0.1 times or more.

The rate of the addition amount of the probe relative to the content of DNAs in a sample may be, for example, a molar ratio relative to double-stranded nucleic acids or a molar ratio relative to single-stranded nucleic acids.

The "melting temperature (Tm)" is usually defined as the temperature at which an increase of an absorbance of a sample at a wavelength of 260nm reaches 50% relative to total increase of the absorbance achievable by increasing temperature of the sample. In general, when double strand nucleic acid, such as a DNA solution, is heated, the absorbance at 260 nm increases. This occurs because of a melting of DNA, which is a phenomenon that a hydrogen bond between both strands of a double strand DNA is loosed by heating, and then the double strand DNA is dissociated to single strand DNA, When all double strand DNA is dissociated and becomes single strand DNA, its absorbance may be about 1.5 times higher than the absorbance at the beginning of heating (absorbance of double strand DNA only), and thereby completion of melting can be determined. Tm is defined based on this phenomenon.

In embodiments, the measurement of the change of the signal for determining a Tm associated with the temperature change may be performed by measuring the absorbance at 260 nm on the basis of the principle as described above. In embodiments, it may be preferable to measure a signal which is based on a signal of a label added to the probe for detecting a polymorphism and which varies depending on the hybridization state of a single-stranded DNA and the probe for detecting a polymorphism. Therefore, it may be preferable to use the fluorescently labeled oligonucleotide as the probe for detecting a polymorphism. Examples of the fluorescently labeled oligonucleotide include a fluorescently labeled oligonucleotide, the fluorescence intensity of which when hybridized to its target sequence being smaller than the fluorescence intensity when not hybridized to its target sequence, and a fluorescently labeled oligonucleotide, the fluorescence intensity of which when hybridized to its target sequence is larger than the fluorescence intensity when not hybridized to its target sequence.

In the case of a probe like the former, the fluorescence signal does not appear or the fluorescence signal is week while the probe forms a hybrid with the sequence to be detected (a double-stranded DNA); and, in other hand, the fluorescence signal appears or the fluorescence signal is increased while the probe is dissociated by heating.

In contrast, in the case of a probe like the latter, the fluorescence signal appears while the probe forms a hybrid with the sequence to be detected (a double-stranded DNA); and the fluorescence signal is decreased (or disappeared) while the probe is dissociated by heating. Therefore, similarly to the measurement of the absorbance at 260 nm as described above, the progress of the melting and a Tm may be determined by detecting the change of the fluorescence signal based on such a fluorescent label under the conditions specific to the fluorescent label (for example, a fluorescence wavelength).

One exemplary embodiment of the method of detecting a polymorphism using the detection the change of the signal based on a fluorescent dye is described by way of a specific example. The method of detecting a polymorphism is characterized by the use of the probe for detecting a polymorphism in itself, and therefore other steps and conditions are not limited in any way.

The sample containing a nucleic acid to be used as a template in a nucleic acid amplification is not particularly limited as long as it contains a nucleic acid. Examples of the sample include samples that are derived from or can be derived from any biological sources, such as blood, a suspension of oral mucosa, somatic cells such as nails and hair, germ cells, milk, ascitic fluid, paraffin-embedded tissue, gastric juice, a gastric washing, peritoneal fluid, amniotic fluid and a cell culture. For the nucleic acid to be used as a template, the source may be used directly or after pretreatment to change the nature of the sample.

Nucleic acids derived from biological samples described above may be isolated, for example, by conventional methods well-known in the art. For example, a commercially available genomic DNA isolation kit (trade name: GFX GENOMIC BLOOD DNA PURIFICATION KIT, available from GE healthcare bioscience) and the like may be utilized to isolate genomic DNA from whole blood.

Next, the probe for detecting a polymorphism containing the fluorescently labeled oligonucleotide is added to a sample containing an isolated genomic DNA.

The probe for detecting a polymorphism may be added to a liquid sample containing an isolated genomic DNA, or may be mixed with a genomic DNA in an appropriate solvent. The solvent is not particularly limited, and examples of the solvent include conventionally known solvents such as a buffer solution such as Tris-HCl, a solvent containing KCl, $MgCl_2$, $MgSO_4$ and/or glycerol, and a PCR reaction solution.

The timing of adding the probes for detecting a polymorphism is not particularly limited, and, for example, in the case where a amplification process such as PCR as described below, the probe may be added to the PCR amplification products after the amplification process or may be added before the amplification process.

When the probe for detecting a polymorphism is added before an amplification process such as PCR, it may be preferable that a fluorescent dye or a phosphate group is added to the 3' end of the probe as described above.

The method of amplifying a nucleic acid is preferably a method that uses a polymerase, and examples of the method include PCR (Polymerase Chain Reaction), ICAN method, LAMP method and NASBA (Nucleic acid sequence based amplification). When the amplification is carried out by a method that uses a polymerase, it may be preferable that the amplification is carried out in the presence of the probe. The conditions of the amplification reaction according to the probe and polymerase that are used may be readiliy adjusted by those of ordinary skill in the art. This may enable to evaluate a polymorphism by analyzing a Tm of the probe after the amplification of the nucleic acid only, and, therefore, it may not be needed to handle the amplification products after the amplification reaction. Therefore, there may be substantially no concern for contamination by the amplification products. In addition, the detection can be performed in the same apparatus as the apparatus required in the amplification. Therefore, it may not be needed to transfer a vessel, and the automatization thereof may be easy.

The pair of primers that is used in PCR is not particularly limited, as long as the primers are capable of amplifying the region to which the probe can hybridize. The pair of primers that is used in PCR may be set in the same manner as in the setting method of a pair of primers in usual PCR. The length and Tm of the primer may be typically in a range of from 12 bases to 40 bases at 40°C to 70°C, and preferably from 16 bases to 30 bases at 55°C to 60°C. The lengths of the two primers in the pair are not needed to be the same, although in embodiments, the Tms of the two primers may be preferably approximately the same (or the difference of the Tms of the two primers may be preferably within 2°C).
One example of a primer set that may be used for amplifying a sequence to be detected in the method of detecting a polymorphism is shown below. This is no more than an exemplary embodiment and therefore the invention is not limited to this.

Table 4

| Name | BASE SEQUENCE | SEQ ID NO. |
|---|---|---|
| MDR1exon21-F primer | AAATGTTGTCTGGACAAGCACTG | SEQ ID NO. 3 |
| MDR1exon21-R primer | AATTAATCAATCATATTTAGTTTGACTCAC | SEQ ID NO. 4 |

As a DNA polymerase that is used in the PCR method, DNA polymerases that are usually used may be used without particular limitation. Examples of the DNA polymerase include GENE TAQ (trade name, manufactured by NIPPON GENE CO., LTD.) and PRIMESTAR MAX DNA POLYMERASE (trade name, manufactured by Takara Bio Inc.).

The PCR may be carried out under the conditions that are appropriately selected from the conditions that are usually used.
During amplification, the amplification may be also monitored by using real-time PCR to measure the copy number of the DNA contained in a sample (a sequence to be detected). In other words, the rate of the probes forming hybrids is increased according to the amplification of the DNA (a sequence to be detected) by PCR, and, due to this, the fluorescence intensity will vary. By monitoring this fluorescence intensity, the copy number and/or abundance ratio of a sequence to be detected (a wild type DNA and/or a mutant type DNA) contained in a sample may be assessed.

In the method of detecting a polymorphism, the fluorescently labeled oligonucleotide and a single-stranded nucleic acid in a sample are made to contact, and thereby the two are hybridized. The single-stranded nucleic acid in a sample may be prepared by, for example, dissociating PCR amplification products obtained as describe above.

The heating temperature in the dissociation of the PCR amplification products (heating temperature in a dissociation) is not particularly limited, as long as it is a temperature wherein the amplification products can be dissociated. For example, the heating temperature may be in a range from 85°C to 95°C. The heating time is also not particularly limited, In embodiments, the heating time may be typically in a range from 1 second to 10 minutes, and preferably in a range from 1 second to 5 minutes.

The hybridization of the dissociated single-stranded DNA and the fluorescently labeled oligonucleotide may be, for example, carried out after the dissociation by reducing the heating temperature in the dissociation. The temperature condition may be, for example, in a range from 40°C to 50°C.

The volume and concentration of each composition in a reaction solution of the hybridization are not particularly limited. In embodiments, the concentration of DNAs in the reaction solution may be, for example, in a range from 0.01 μM to 1 μM, and preferably in a range from 0.1 μM to 0.5 μM. The concentration of the fluorescently labeled oligonucleotide may be, for example, a range that satisfies the above-described ratio of the addition amount relative to DNAs is preferable, and it may be, for example, in a range from 0.001 μM to 10 μM, and preferably in a range from 0.001 μM to 1 μM.

The thus-obtained hybrid of the single-stranded DNA and the fluorescently labeled oligonucleotide is then gradually heated to measure the change of the fluorescence signal associated with the temperature increase. For example, in the case where Q PROBE® is used, the fluorescence intensity is decreased (or quenched) in a state in which the probe is hybridized with a single-stranded DNA, as compared to the fluorescence intensity in a state in which the probe is dissociated. Therefore, in this case, the measurement of the change of the fluorescence intensity may be, for example, carried out by gradually heating a hybrid the fluorescence of which being decreased (or quenched) and measuring the increase of the fluorescence intensity associated with the temperature increase.

The range of the temperature while measuring the change of the fluorescence intensity is not particularly limited. For example, the start temperature may be in a range from room temperature to 85°C, preferably in a range from 25°C to 70°C, and the end temperature may be, for example, in a range from 40°C to 105°C. In addition, the temperature increasing rate is also not particularly limited. For example, the temperature increasing rate is in a range from 0.1°C/second to 20°C/second, preferably in a range from 0.3°C/second to 5°C/second.

Next, the change of the signal is analyzed to determine the Tm. More specifically, the Tm may be determined by calculating a differential value at each temperature from the obtained fluorescence intensity (-d(Fluorescence Intensity)/dt) and considering a temperature wherein the differential value is the lowest as a Tm. Alternatively, the Tm may also be determined by considering a point wherein an amount of the increase in the fluorescence intensity per unit time ((Amount of Increase in Fluorescence Intensity)/t) is the highest as a Tm. On the contrary, in the case where a probe the signal intensity of which being increased by hybridization is used as a labeled probe instead of the quenching probe, the signal analysis and the determination of a Tm can be carried out by measuring an amount of the decrease in the fluorescence intensity.

As described in above, signal change caused by increase of the temperature, which may be preferably an increase of the fluorescence intensity, can be measured by increasing a temperature of a reaction solution which contains the hybrid, i.e. by heating a hybrid. Alternatively, for example, signal change caused by hybridization can be measured. That is, when decreasing a temperature of a reaction solution which contains the probe to form a hybrid, signal change caused by decrease of the temperature can be measured.

As a specific example, in the case where a fluorescently labeled oligonucleotide probe whose fluorescence intensity when hybridizing to its target sequence is decreased (quenched) as compared to the fluorescence intensity when not hybridizing to its target sequence (for example, a Q PROBE®) is used, the fluorescence intensity is high at the time when the probe is added to a sample since the probe is in a state of dissociation, but, when hybrids are formed by temperature reduction, the fluorescence is decreased (or quenched). Therefore, the measurement of the change of the fluorescence intensity may be carried out by, for example, gradually reducing the temperature of the heated sample and measuring the decrease of the fluorescence intensity associated with the temperature reduction.

On the other hand, in the case where a labeled probe whose signal is increased by hybridization is used, the fluorescence intensity is low (or quenched) at the time when the probe is added to a sample since the probe is in a state of dissociation, but, when hybrids are formed by temperature reduction, the fluorescence intensity is increased. Therefore, the measurement of the change of the fluorescence intensity can be carried out by gradually reducing the temperature of the sample and measuring the increase of the fluorescence intensity associated with the temperature reduction, for example.

The method of detecting a polymorphism is characterized in that at least one of the fluorescently labeled oligonucleotides that are capable of detecting a mutation in the exon 21 of the MDR1 gene (the specific fluorescently labeled oligonucleotide) is used. In addition to this specific fluorescently labeled oligonucleotide, it may be preferable to simultaneously use a third fluorescently labeled oligonucleotide, that is capable of detecting a mutation in the exon 26 of the MDR1 gene. By detecting both mutations in the exon 21 and in the exon 26, for example, the expression amount of P-glycoprotein derived from the MDR1 gene may be estimated with a better accuracy.

The third fluorescently labeled oligonucleotide is not particularly limited, as long as it is capable of detecting a mutation of the 256th base of the base sequence of SEQ ID NO: 1. The third fluorescently labeled oligonucleotide may be constructed in the similar manner as in the specific fluorescently labeled oligonucleotide as described above. For example, a fluorescently labeled oligonucleotide as described in JP-A No. 2005-28733 may be herein used.

The base sequence of SEQ ID NO: 1 is a partial sequence of the exon 26 of the MDR1 gene, and the 3435th base in the exon 26 corresponds to the 256th base of the base sequence of SEQ ID NO: 1.

Specific examples of the third fluorescently labeled oligonucleotide include a fluorescently labeled oligonucleotide having a sequence complementary to a base sequence having a length of from 14 bases to 50 bases that starts from the 243rd base in the base sequence of SEQ ID NO: 1, and a fluorescently labeled oligonucleotide having a sequence complementary to base sequence having a length of from 9 bases to 50 bases that starts from the 248th base of the base sequence of SEQ ID NO: 1.

The third fluorescently labeled oligonucleotide may be preferably a fluorescently labeled oligonucleotide having a base sequence complementary to a base sequence having a length of from 9 bases to 50 bases that starts from the 248th base of the base sequence of SEQ ID NO: 1, and more preferably a fluorescently labeled oligonucleotide having a base sequence complementary to a base sequence having a length of from 9 bases to 50 bases that starts from the 248th base of the base sequence of SEQ ID NO: 1, and having a fluorescent dye at its 5' end. When the third fluorescently labeled oligonucleotide is a fluorescently labeled oligonucleotide like these, for example, the sensitivity for detecting a mutation may be further improved.

Specific examples of a probe which has the above-described base sequence and having a cytosine at its 5'- or 3'- end which is labeled with a fluorescent dye include the one shown in the following Table 5, in which the base described by a capital letter each represents a mutated site, and the P each represents a phosphate group. Note that the fluorescently labeled oligonucleotide is not limited to this example.

Table 5

| Name | BASE SEQUENCE | Length | SEQ ID NO. |
|---|---|---|---|
| MDR1exon26_probe | (Pacific Blue)-ctgccctcacAatctcttc-P | 19 | SEQ ID NO. 7 |

The change of the fluorescence signal caused by the third fluorescently labeled oligonucleotide may be measured in the similar manner as in the specific fluorescently labeled oligonucleotide.

When the third fluorescently labeled oligonucleotide is used, the fluorescent dye contained in the third fluorescently labeled oligonucleotide may be preferably a fluorescent dye the fluorescence emission wavelength of which being different from that of the fluorescent dye contained in the specific fluorescently labeled oligonucleotide. Thereby, the change of the fluorescence signal caused by the specific fluorescently labeled oligonucleotide and the change of the fluorescence signal caused by the third fluorescently labeled oligonucleotide can be measured at the same time.

In the case where the third fluorescently labeled oligonucleotide is used in combination with the specific fluorescently labeled oligonucleotide in the method of detecting a polymorphism, it may be preferable that the method further includes performing amplification by using, as a template, a region in the base sequence of SEQ ID NO: 1, in which the region includes the 256th base of the base sequence of SEQ ID NO:1 and having a length of from 50 bases to 1000 bases. The length of the region may be more preferably from 80 bases to 200 bases. This may enable, for example, to detect a mutation in the exon 26 of the MDR1 gene with a higher sensitivity.

The method of amplifying the region which contains the 256th base of the base sequence of SEQ ID NO: 1 and having a length of from 50 bases to 1000 bases is not particularly limited. For example, the PCR as described above may be used.

The primers that are applied to PCR are not particularly limited, as long as they are capable of amplifying the above-described region that contains the 256th base of interest in the base sequence of SEQ ID NO: 1. For example, primers described in JP-A No. 2005-287335 may also be applied.

Examples of a primer set that may be used for amplifying a region containing the 256th base of the base sequence of SEQ ID NO: 1 in the method of detecting a polymorphism include one having the primers shown below.

Table 6

| Name | BASE SEQUENCE | SEQ ID NO. |
|---|---|---|
| MDR1exon26-F primer | AGTGCAGCATTGCTGAGAAG | SEQ ID NO. 5 |
| MDR1exon26-R primer | CAGAGAGGGTGCCACATGCTC | SEQ ID NO .6 |

In the method of detecting a polymorphism, the type of the bases at the mutation site can be identified by using, as probes for detecting polymorphism, at least two fluorescently labeled oligonucleotides which are different from each other in terms of bases complementary to the 300th base of the base sequence of SEQ ID NO:2 (bases at the mutation site) by independently having a C, a T or an A as the bases.

For example, a method for identifying the base at the mutation site in the exon 21 of the MDR1 gene by using a "probe 1", which is a fluorescently labeled P1 oligonucleotide that has the base sequence of SEQ ID NO:8 and has a fluorescent dye (for example, TAMRA (described above)) at its 3' end and a "probe 2", which is a fluorescently labeled P2 oligonucleotide that has the base sequence of SEQ ID NO:9 and has a fluorescent dye (for example, BODIPY FL (described above)) at its 5' end, is described below with reference to the drawings.

The probe 1 is complementary to the wild-type base sequence (in other words, complementary to the base sequence in which a base at the mutation site is a G), and the probe 2 is complementary to the base sequence in which base at the mutation site is a T.

Examples of differential melting curves obtained by using various DNAs to be detected are each shown in FIGs. 2A to 7B. In these figures, the abscissa represents the temperature and the ordinate represents the differential value of the fluorescence intensity; and, each of the figures titled with the letter "A" shows a differential melting curve at the measurement wavelength of the fluorescent dye of the probe 2, and each of the figures titled with the letter "B" shows a differential melting curve at the measurement wavelength of the fluorescent dye of the probe 1.

A method for identifying the type of a mutation of a DNA in a sample by confirming the relationship between a peak position of the melting curve of the sample and the Tm of the wild type or the T-type mutant type in the method of detecting a polymorphism is specifically described below.

That is, when the peak of the melting curve matches the Tm of the wild type, it can be found that the DNA in the sample contains the wild-type base. On the other hand, when the peak of the melting curve matches the Tm corresponding to the T type mutation, it can be found that the DNA in the sample contains the T-type mutation. When the peak of the melting curve matches neither the Tm of the wild type nor the Tm of the T-type mutation, it can be found that the DNA in the sample contains only the A-type mutation, that is neither the wild-type nor the T-type mutation. In addition, when the number of the peak of the melting curve is one, it means that the DNA in the sample contains either one of the wild-type base and the T-type mutation; and, when the number of the peak of the melting curve is two, it can be found that the DNA in the sample is a hetero type that contains both of the wild-type base and the T-type mutation. The mutation (s) in a DNA in a sample may be found by applying the approach combining these information.

FIGs. 2A and 2B show examples of the differential melting curves in the case where the DNA to be detected is a wild type (in other words, the base at the mutation site is a G). The peak of the change of the fluorescence intensity matches the Tm corresponding to the wild type (the Tm at the position shown by a broken line in FIG. 2B) in FIG. 2B. Therefore, it is found, by applying the approach, that the DNA to be detected contains the wild-type base. In addition, only one peak of the change of the fluorescence intensity appears and the temperature of the peak is lower than the Tm in the case where the base at the mutation site is a T (the Tm at the position shown by a broken line in FIG. 2A) in FIG. 2A. Therefore, it is found that the DNA to be detected contains the wild-type base only as the base at the mutation site.

Similarly, by applying the approach, it is found that: the melting curves in FIGs. 3A and 3B show the case where a mutation contained in the DNA to be detected is the A-type mutation only; the melting curves in FIGs. 4A and 4B show the case where the DNA to be detected contains the T-type mutation only as the base at the mutation site; the melting curves in FIGs. 5A and 5B show the case where the wild type and the A-type mutation are contained; the melting curves in FIGs. 6A and 6B show the case where the wild type and the T-type mutation are contained; and the melting curves in FIGs. 7A and 7B show the case where the A-type mutation and the T-type mutation are contained.

As described above, the type of the bases at the mutation site can be identified easily with a high sensitivity by using, as probes for detecting polymorphism, at least two fluorescently labeled oligonucleotides which are different from each other in terms of bases complementary to the 300th base of the base sequence of SEQ ID NO:2 (bases at the mutation site) and have a C, a T or an A as the complementary bases.

The method of detecting a mutation of the 256th base of the base sequence of SEQ ID NO: 1 in the method of detecting a polymorphism is described below with reference to the drawings.

For example, the C3435T mutation in the exon 26 of the MDR1 gene can be detected by using the third fluorescently labeled oligonucleotide that has the base sequence of SEQ ID NO:7 and has a fluorescent dye (for example, PACIFIC BLUE (described above)) at its 5' end (hereinafter also referred to as "the exon26 probe").

The exon26 probe is complementary to the base sequence in which a base at the mutation site is a thymine (T).

FIG. 8 shows an example of the differential melting curve in the case where the DNA to be detected is a wild type. In FIG. 8, since the temperature of the peak of the change of the fluorescence intensity is lower than the Tm corresponding to the T-type mutant type (the Tm at the position shown by a broken line in FIG. 8), it is found that the DNA to be detected contains the wild-type base.

FIG. 9 shows an example of the differential melting curve in the case where the DNA to be detected is the T-type mutant type. In FIG. 9, since the temperature of the peak of the change of the fluorescence intensity is at the Tm corresponding to the T-type mutant type (the Tm at the position shown by a broken line in FIG. 9), it is found that the DNA to be detected contains the wild-type base.

FIG. 10 shows an example of the differential melting curve in the case where the DNA to be detected is a hetero type of the T-type mutant type and a wild type. In FIG. 10, since the temperatures of the peaks of the change of the fluorescence intensity are both at the Tm corresponding to the T-type mutant type and at a temperature lower than the Tm corresponding to the T-type mutant type (the Tm at the position shown by a broken line in FIG. 10), it is found that the DNA to be detected contains the wild-type base.

Method of Evaluating the Efficacy of Drug

According to the method of detecting a polymorphism in the MDR1 gene of this embodiment, whether a mutation exists in the exon 21 of the MDR1 gene or not and the type of the bases at the mutation site may be detected; and, in addition to this, whether a mutation exists in the exon 26 or not may also be detected at the same time. By using this method, the resistance of a source of a sample to a drug and/or the efficacy of a drug may be evaluated on the basis of the absence or presence of the polymorphism(s) and/or the abundance ratio of the mutant sequence(s) and/or the normal sequence(s). The method of evaluating the efficacy of a drug of this embodiment may be useful for, for example, deciding on the basis of the absence or presence of the mutation(s) and/or the abundance ratio of the mutant sequence (s) whether the therapeutic strategy of a disease should be shifted so as to increase the dosage of the drug or use other therapeutic agent instead of the drug.

Kit for Detecting Polymorphism

The kit for detecting a polymorphism of this embodiment is constructed by including a probe for detecting a polymorphism that contains at least one of the specific fluorescently labeled oligonucleotides. In embodiments, the kit may further include other components such as a primer. The kit for detecting a polymorphism is capable of detecting the mutation in the exon 21 of the MDR1 gene.

The probe for detecting a polymorphism may be a probe that contains one fluorescently labeled oligonucleotide, or may be a probe that contains two or more fluorescently labeled oligonucleotides. In embodiments, it may be preferable to contain at least two fluorescently labeled oligonucleotides which are different from each other in terms of bases complementary to the 300th base of the base sequence of SEQ ID NO: 2 and have a C, a T or an A as the complementary bases; and it is more preferable to further contain at least one of the probe for detecting a polymorphism which is capable of detecting a mutation of the 256th base of the base sequence of SEQ ID NO: 1.

When two or more fluorescently labeled oligonucleotides are contained as the probe for detecting a polymorphism, the fluorescently labeled oligonucleotides may be contained in a combination form, or may be independently contained.

In addition, in the case where the kit for detecting a polymorphism contains two or more of the probes in a mixed form; and in the case where two or more of the probes are independently contained as a separate reagent but are to be used in the same reaction system, for example, for carrying out the Tm analysis between each fluorescently labeled oligonucleotide and each sequence to be detected, the fluorescence emission wavelengths of the fluorescent dyes with which the two or more probes are labeled may be preferably different from each other.

By using different fluorescent substances as described above, even in the case where two or more probes are used in the same reaction system, the detection for the two or more probes may be carried out at the same time.

In addition to the probe, the kit for a polymorphism may further contain reagents that are required for carrying out the nucleic acid amplification in the method of detecting a polymorphism, especially a primer for the amplification using a DNA polymerase. The probe, primers and other reagents may be independently accommodated in the kit, or some of them may be accommodated in the kit as a mixture.

It may be preferable that the kit for detecting a polymorphism(s) further contains a primer that is capable of performing amplification by using, as a template, a region that is in the base sequence of SEQ ID NO: 2 and includes at least a sequence to which the P1 oligonucleotide or the P2 oligonucleotide hybridizes.

Further, when the kit for detecting a polymorphism(s) contains the probe for detecting a polymorphism which is capable of detecting the mutation of the 256th base of the base sequence of SEQ ID NO: 1, it may be preferable that the probe includes at least a fluorescently labeled oligonucleotide having a base sequence that is complementary to a sequence having a length of from 9 bases to 50 bases that starts from the 248th base of the base sequence of SEQ ID NO: 1, and it maybe preferable that the kit further contains a primer that is capable of performing amplification by using, as a template, a region in the base sequence of SEQ ID NO: 1, comprising the 256th base of the base sequence of SEQ ID NO: 1, and having a length of from 50 bases to 1000 bases, which is more preferably from 80 bases to 200 bases.

By including a primer set for amplifying a sequence that contains such polymorphism site (a region to which the probe (s) hybridize(s)), for example, the polymorphism can be detected with a higher sensitivity.

Further, it may be preferable that the kit for detecting a polymorphism further contain an instruction that describes the detection process which includes: forming a differential melting curve for the nucleic acid to be detected that is contained in the sample by using the probe for detecting a polymorphism; and then carrying out the analysis of the Tm to detect a mutation(s) in the MDR1 gene.

EXAMPLES

In the following, the invention is described in further detail with reference to examples. However, the examples are not be construed as limiting the invention.

Example 1

PCR and Tm analysis were carried out using a fully-automated SNP analyzer (I-DENSY (trademark), manufactured by ARKRAY, Inc.) and the reagents for examination of the formulation as shown in Table 7.

The PCR condition was one process of 95°C for 60 seconds, and then repeating 50 cycles of 95°C for 1 second and 58°C for 30 seconds.

The Tm analysis was carried out after the PCR by one process of 95°C for 1 minute and at 40°C for 60 seconds, and then measuring the change of the fluorescence intensity over time while increasing the temperature from 40°C to 75°C at a temperature increasing rate of 1°C per 3 seconds. By using measurement wavelengths in a range from 585 nm to 700 nm and in a range from 520 nm to 555 nm, the change of the fluorescence intensities derived from the MDR1 exon21 probe 1 and the MDR1 exon21 probe 2 were measured respectively.

Table 7

| Formulation (μl) | |
|---|---|
| $H_2O$ | 22.69 |
| 0.94U/μl Taq Pol | 2 |
| 100mM $MgCl_2$ | 0.75 |
| 1M KCl | 1.25 |
| 1M Tris-HCl (pH8.6) | 1.25 |
| 2.5mMdNTP | 4 |
| 20w/vol[%] BSA | 0.5 |
| 80vol/vol[%] Glycerol | 1.56 |
| 5μM MDR1 exon26 probe | 4 |
| 5μM MDR1 exon21 probe 1 | 4 |
| 5μM MDR1 exon21 probe 2 | 4 |
| 100μM MDR1 exon21-F primer | 1 |
| 100μM MDR1 exon21-R primer | 0.5 |
| 100μM MDR1 exon26-F primer | 1 |
| 100μM MDR1 exon26-R primer | 0.5 |
| Sample | 1 |
| | 50 |

Details of the primers and probes in Table 8 are shown below. The "P" in the probe indicates that the probe is phosphorylated at its 3' end.

Table 8

| Name | BASE SEQUENCE | SEQ ID NO. |
|---|---|---|
| MDR1exon26_probe | (Pacific Blue)-ctgccctcacAatctcttc-P | SEQ ID NO. 7 |
| MDR1exon21_probe 1 | cttcccagCaccttctagttc-(TAMRA) | SEQ ID NO. 8 |
| MDR1exon21_probe 2 | cccagAaccttctagttc | SEQ ID NO. 9 |
| MDR1exon21-F primer | AAATGTTGTCTGGACAAGCACTG | SEQ ID NO. 3 |
| MDR1exon21-R primer | AATTAATCAATCATATTTAGTTTGACTCAC | SEQ ID NO. 4 |
| MDR1exon26-F primer | ACTGCAGCATTGCTGAGAAC | SEQ ID NO. 5 |
| MDR1exon26-R primer | CAGAGAGGCTGCCACATGCTC | SEQ ID NO. 6 |

A human genome purified from the whole blood was used in an amount of 1 μl (100 cp/μl) as a sample having a mutation(s) to be detected. The human genome that was used is one in that the 2677th base in the exon 21 of its MDR1 gene is a wild-type base (G). The obtained differential melting curves are shown in FIGs. 2A and 2B. FIG. 2A shows a differential melting curve obtained by using the MDR1 exon21 probe 2, and FIG. 2B shows a differential melting curve obtained by using the MDR1 exon21 probe 1.
From FIGs. 2A and 2B, it is found that the 2677th base in the exon 21 of the MDR1 gene contained in the sample to be detected is a wild-type base.

Example 2

Tm analysis was carried out in the similar manner as in Example 1, except that 1 μl of a 25 μM artificial oligonucleotide which has a base length of 50 bases and has a mutation to be detected and has a base sequence in which the 2677th base in the exon 21 of the MDR1 had been mutated into an adenine (A) was used as a sample, and that PCR was not carried out. The obtained differential melting curves are shown in FIGs. 3A and 3B. FIG. 3A shows a differential melting curve obtained by using the MDR1 exon21 probe 2, and FIG. 3B shows a differential melting curve obtained by using the MDR1 exon21 probe 1.
The obtained differential melting curves corresponded to those in the case where the 2677th base in the exon 21 of the MDR1 gene contained in the sample to be detected is the prescribed mutant type.

Example 3

A human genome purified from the whole blood was used in an amount of 1 μl (100 cp/μl) as a sample having a mutation(s) to be detected. The human genome that was used is one in that the 2677th base in the exon 21 of its MDR1 gene is a mutant-type base of thymine (T). Tm analysis was carried out in the similar manner as in Example 1, except that the human genome having the mutant-type base of thymine (T) as the 2677th base in the exon 21 of its MDR1 gene was used as a sample having mutation(s) to be detected. The obtained differential melting curves are shown in FIGs. 4A and 4B. FIG. 4A shows a differential melting curve obtained by using the MDR1 exon21 probe 2, and FIG. 4B shows a differential melting curve obtained by using the MDR1 exon21 probe 1.
The obtained differential melting curves corresponded to those in the case where the 2677th base in the exon 21 of the MDR1 gene contained in the sample to be detected is the prescribed mutant type.

Example 4

Tm analysis was carried out in the similar manner as in Example 1, except that 1 μl of a 25 μM nucleic acid mixture and that PCR was not carried out. Herein, the nucleic acid mixture contains a wilt-type artificial oligonucleotide having a length of 50 bases and a mutant-type artificial oligonucleotide having a length of 50 bases and having a sequence in which the 2677th base in the exon 21 of its MDR1 gene is mutated into an adenine (A) at a mixing ratio of 1:1. The obtained differential melting curves are shown in FIGs. 5A and 5B. FIG. 5A shows a differential melting curve obtained by using the MDR1 exon21 probe 2, and FIG. 5B shows a differential melting curve obtained by using the MDR1 exon21 probe 1.
The obtained differential melting curves corresponded to those in the case where the 2677th base in the exon 21 of the MDR1 gene contained in the sample to be detected is the prescribed mutant type.

Example 5

A human genome purified from the whole blood was used in an amount of 1 μl (100 cp/μl) as a sample having a mutation(s) to be detected. Tm analysis was carried out in the similar manner as in Example 1, except that the human genome having the hetero mutant-type base of guanine (G) and thymine (T) as the 2677th base in the exon 21 of its MDR1 gene was used as a sample having mutation(s) to be detected. The obtained differential melting curves are shown in FIGs. 6A and 6B. FIG. 6A shows a differential melting curve obtained by using the MDR1 exon21 probe 2, and FIG. 6B shows a differential melting curve obtained by using the MDR1 exon21 probe 1.
The obtained differential melting curves corresponded to those in the case where the 2677th base in the exon 21 of the MDR1 gene contained in the sample to be detected is the prescribed mutant type.

Example 6

A human genome purified from the whole blood was used in an amount of 1 μl (100 cp/μl) as a sample having a mutation(s) to be detected. Tm analysis was carried out in the similar manner as in Example 1, except that the human genome having the hetero mutant-type base of adenine (A) and thymine (T) as the 2677th base in the exon 21 of its MDR1 gene was used as a sample having inutation(s) to be detected. The obtained differential melting curves are shown in FIGs. 7A and 7B. FIG. 7A shows a differential melting curve obtained by using the MDR1 exon21 probe 2, and FIG 7B shows a differential melting curve obtained by using the MDR1 exon21 probe 1.
The obtained differential melting curves corresponded to those in the case where the 2677th base in the exon 21 of the MDR1 gene contained in the sample to be detected is the prescribed mutant type.

Example 7

A human genome purified from the whole blood was used in an amount of 1 μl (100 cp/μl) as a sample having a mutation(s) to be detected. Tm analysis was carried out in the similar manner as in Example 1, except that the human genome having the wilt-type base as the 3435th base in the exon 26 of its MDR1 gene was used as a sample, and the measurement wavelengths of from 445 nm to 480 nm was used. The differential melting curve corresponding to the MDR1 exon26 probe among those obtained for this test is shown in FIG. 8.
The obtained differential melting curve corresponded to that in the case where the 3435th base in the exon 26 of the MDR1 gene contained in the sample to be detected is the wild type.

Example 8

Tm analysis was carried out in the similar manner as in Example 7, except that 1 μl of a 25 μM artificial oligonucleotide which has a base length of 50 bases and has a mutation to be detected and has a base sequence in which the 3435th base in the exon 26 of the MDR1 had been mutated into a thymine (T) was used as a sample, and that PCR was not carried out. The differential melting curve corresponding to the MDR1 exon26 probe among those obtained for this test is shown in FIG. 9.
The obtained differential melting curve corresponded to that in the case where the 3435th base in the exon 26 of the MDR1 gene contained in the sample to be detected is the prescribed mutant type.

Example 9

A human genome purified from the whole blood was used in an amount of 1 μl (100 cp/μl) as a sample having a mutation(s) to be detected. Tm analysis was carried out in the similar manner as in Example 7, except that the human genome having the hetero mutant-type base of cytosine (C) and thymine (T) as the 3435th base in the exon 26 of the MDR1 gene was used as a sample having mutation(s) to be detected. The differential melting curve corresponding to the MDR1 exon26 probe among those obtained for this test is shown in FIG. 10.
The obtained differential melting curve corresponded to that in the case where the 3435th base in the exon 26 of the MDR1 gene contained in the sample to be detected is the prescribed mutant type.

Comparative example 1

Measurement of the change of the fluorescence intensity was carried out in the similar manner as Example 1, except that only a fluorescently labelled oligonucleotide MDR1 exon21 probe 3, that has a base sequence shown below, was used as a fluorescently labelled oligonucleotide.
The differential melting curve obtained thereby had too small changes to evaluate Tm therefrom.
Table 9

| Name | BASE SEQUENCE | SEQ ID NO. |
|---|---|---|
| MDR1exon21_probe 3 | (TAMURA)-ctagaaggttctgggaag-P | SEQ ID NO. 10 |

It is understood from the above that the G2677A/T mutation in the exon 21 of the MDR1 gene may be detected with a high sensitivity and easily by using the polymorphism detection probe of one aspect of the invention. It is further understood that the C3435T mutation in the exon 26 of the MDR1 gene may also be detected at the same time by simultaneously using, in addition to the probe, a probe for detecting a polymorphism that is capable of detecting a mutation of the 256th base of the base sequence of SEQ ID NO: 1.
All publications, patent applications, and technical standards mentioned in this specification are herein incorporated by reference to the same extent as if such individual publication, patent application, or technical standard was specifically and individually indicated to be incorporated by reference. It may be obvious to those having skill in the art that many changes may be made in the above-described details of the preferable embodiments of the present invention. It is intended that the scope of the invention be defined by the following claims and their equivalents.

## Claims

1. A probe which detects a polymorphism in the MDR1 gene, the probe comprising at least one fluorescently labeled oligonucleotide selected from the group consisting of a P1 oligonucleotide and a P2 oligonucleotide,
the P1 oligonucleotide having (1) a sequence that is complementary to a first base sequence or (2) a sequence that is homologous to (1), the first base sequence being a partial sequence of SEQ ID NO:2 having a length of 13 bases to 68 bases and comprising the 288th to 300th bases of SEQ ID NO:2, and the P1 oligonucleotide having, as a base complementary to the 288th base, a base that is labeled with a first fluorescent dye, and
the P2 oligonucleotide having (3) a sequence that is complementary to a second base sequence or (4) a sequence that is homologous to (3), the second base sequence being a partial sequence of SEQ ID NO:2 having a length of 6 bases to 93 bases and comprising the 300th to 305th bases of SEQ ID NO:2, and the P2 oligonucleotide having, as a base complementary to the 305th base, a base that is labeled with a second fluorescent dye.

2. The probe of claim 1, wherein the base labeled with the first fluorescent dye is at a position of any one of 1st to 3rd positions from the 3' end of the P1 oligonucleotide, and the base labeled with the second fluorescent dye is at a position of any one of 1 st to 3rd positions from the 5' end of the P2 oligonucleotide, preferably wherein the base labeled with the first fluorescent dye is at the 3' end of the P1 oligonucleotide, and the base labeled with the second fluorescent dye is at the 5' end of the P2 oligonucleotide.

3. The probe of claim 1 or 2, wherein the fluorescence intensity of the fluorescently labeled oligonucleotide when hybridized to its target sequence is larger or smaller than the fluorescence intensity when not hybridized to its target sequence.

4. The probe of any one of claims 1 to 3, wherein the fluorescence intensity of the fluorescently labeled oligonucleotide when hybridized to its target sequence is smaller than the fluorescence intensity when not hybridized to its target sequence.

5. The probe of any one of claims 1 to 4, wherein the length of the P1 oligonucleotide is in a range of 13 bases to 56 bases and the length of the P2 oligonucleotide is in a range of 6 bases to 29 bases, preferably
wherein the length of the P1 oligonucleotide is in a range of 13 bases to 26 bases and the length of the P2 oligonucleotide is in a range of 6 bases to 23 bases, further preferably wherein the length of the P1 oligonucleotide is in a range of 13 bases to 21 bases and the length of the P2 oligonucleotide is in a range of 6 bases to 18 bases.

6. The probe of any one of claims 1 to 5, being a probe for melting curve analysis.

7. The probe of any one of claims 1 to 6, comprising at least two fluorescently labeled oligonucleotides that are different from each other in terms of bases complementary to the 300th base of the base sequence of SEQ ID NO:2 and have a C, a T or an A as the complementary bases.

8. A method of detecting a polymorphism of the MDR1 gene, the method comprising using the probe of any one of claims 1 to 7.

**9.** The method of claim 8, comprising:

(I) obtaining a hybrid formed of a single-stranded nucleic acid and the probe by hybridizing the fluorescently labeled oligonucleotide and the single-stranded nucleic acid by contacting the single-stranded nucleic acid in a sample with the probe;
(II) measuring a change in a signal based on dissociation of the hybrid by changing the temperature of the sample comprising the hybrid in order to dissociate the hybrid;
(III) determining, as a melting temperature, a temperature at which the hybrid dissociates based on the signal variation; and
(IV) checking for the presence of the polymorphism of the MDR1 gene based on the melting temperature.

**10.** The method of claim 8 or 9, further comprising obtaining the single-stranded nucleic acid by performing amplification of a nucleic acid before the (I) obtaining a hybrid or during the (I) obtaining a hybrid.

**11.** The method of any one of claims 8 to 10, further comprising contacting a probe with the single-stranded nucleic acid in the sample, the probe being capable of detecting a mutation of the 256th base of the base sequence of SEQ ID NO:1,
wherein preferably the probe that is capable of detecting a mutation of the 256th base of the base sequence of SEQ ID NO:1 is a fluorescently labeled oligonucleotide having a base sequence that is complementary to a sequence having a length of 9 bases to 50 bases that starts from the 248th base of the base sequence of SEQ ID NO:1.

**12.** A method of evaluating a drug, comprising:

detecting a polymorphism in the MDR1 gene by the method of any one of claims 8 to 11; and
evaluating resistance of a source of the sample to the drug or an effect of the drug based on a result of the detection.

**13.** A kit for detecting a polymorphism, comprising the probe of any one of claims 1 to 7.

**14.** The kit of claim 13, further comprising a primer that is capable of performing amplification by using, as a template, a region that is in the base sequence of SEQ ID NO:2 and comprises a sequence to which the P1 oligonucleotide or the P2 oligonucleotide hybridizes.

**15.** The kit of claim 13 or 14, further comprising a fluorescently labeled oligonucleotide having a base sequence that is complementary to a sequence having a length of 9 bases to 50 bases that starts from the 248th base of the base sequence of SEQ ID NO:1.

FIG. 1A

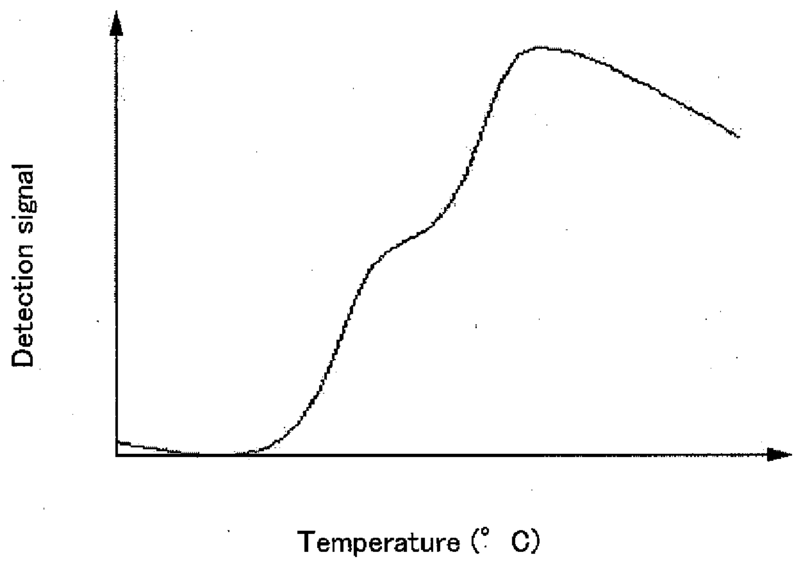
Detection signal
Temperature (° C)

FIG. 1B

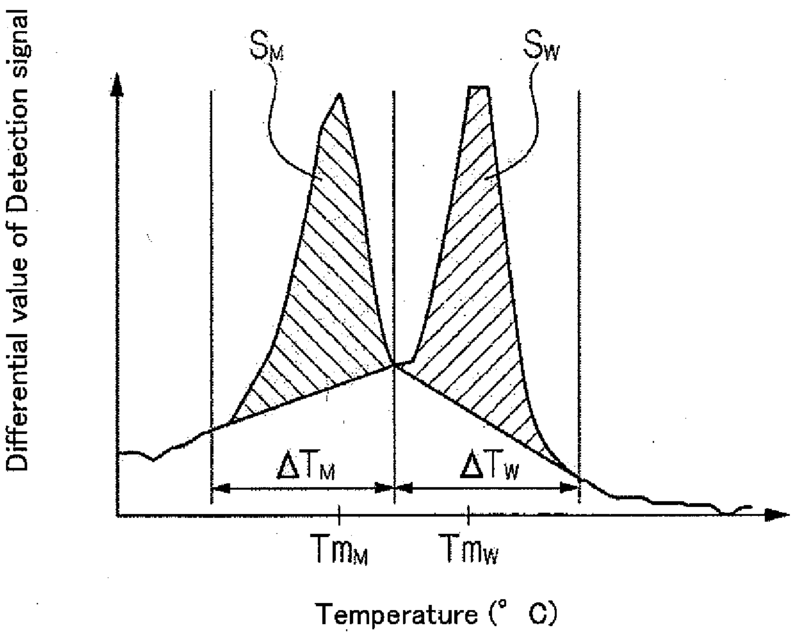
Differential value of Detection signal
$S_M$
$S_W$
$\Delta T_M$
$\Delta T_W$
$Tm_M$
$Tm_W$
Temperature (° C)

FIG. 2A

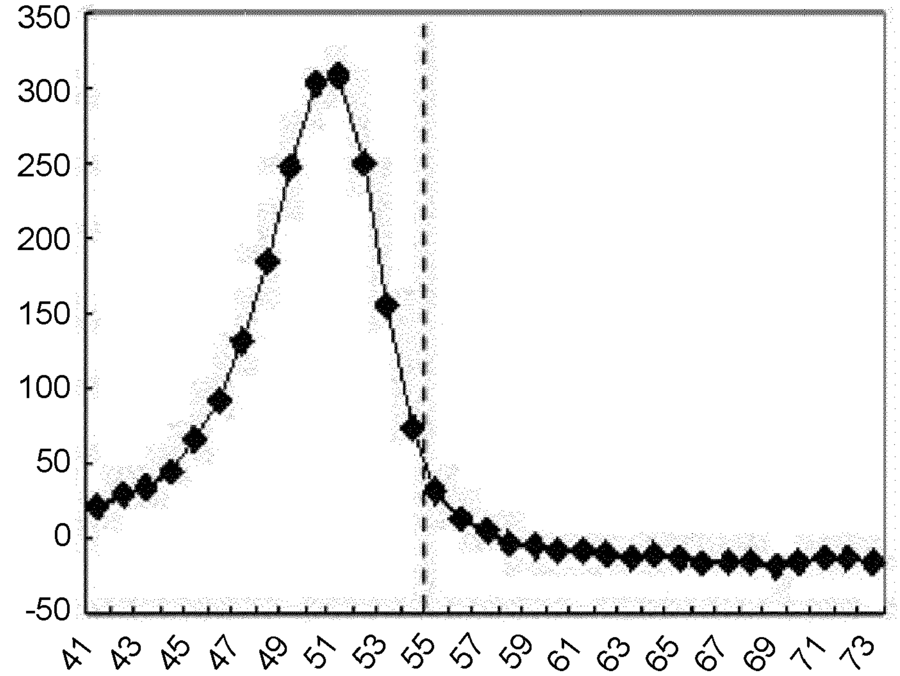
350
300
250
200
150
100
50
0
-50
41
43
45
47
49
51
53
55
57
59
61
63
65
67
69
71
73

FIG. 2B

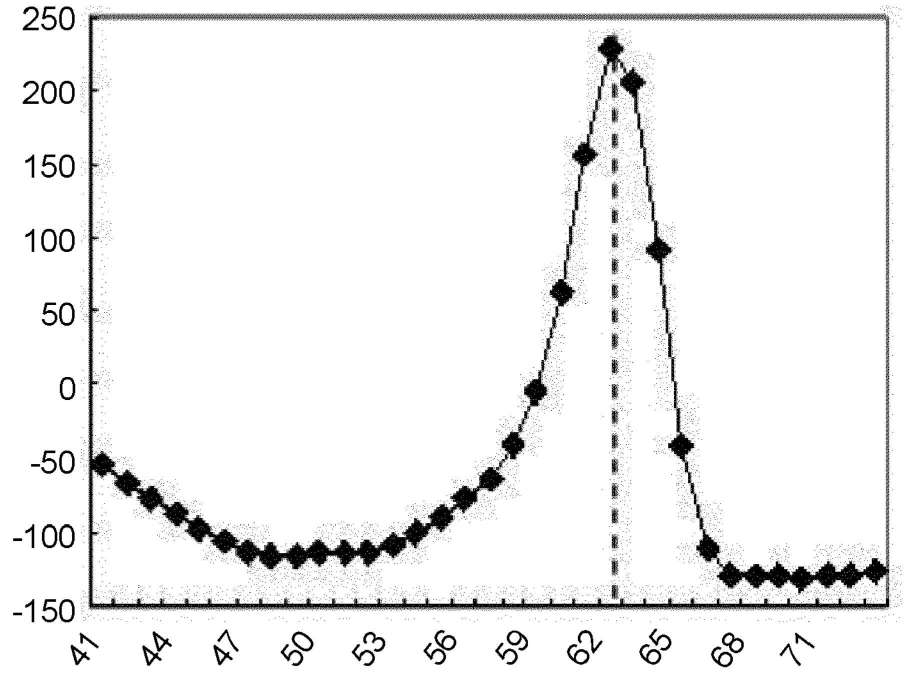
250
200
150
100
50
0
-50
-100
-150
41
44
47
50
53
56
59
62
65
68
71

FIG. 3A

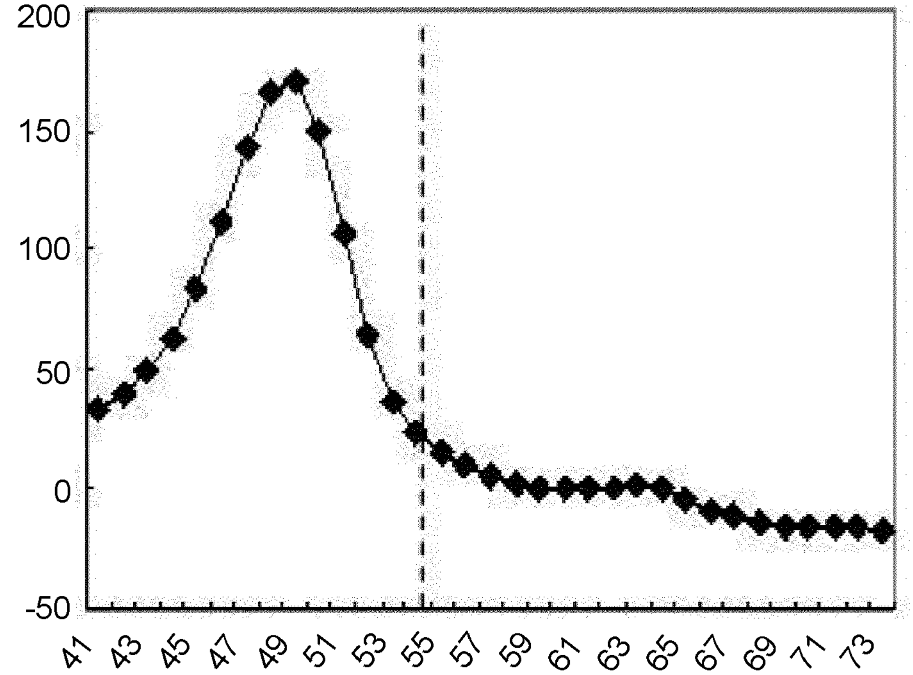
200
150
100
50
0
-50
41
43
45
47
49
51
53
55
57
59
61
63
65
67
69
71
73

FIG. 3B

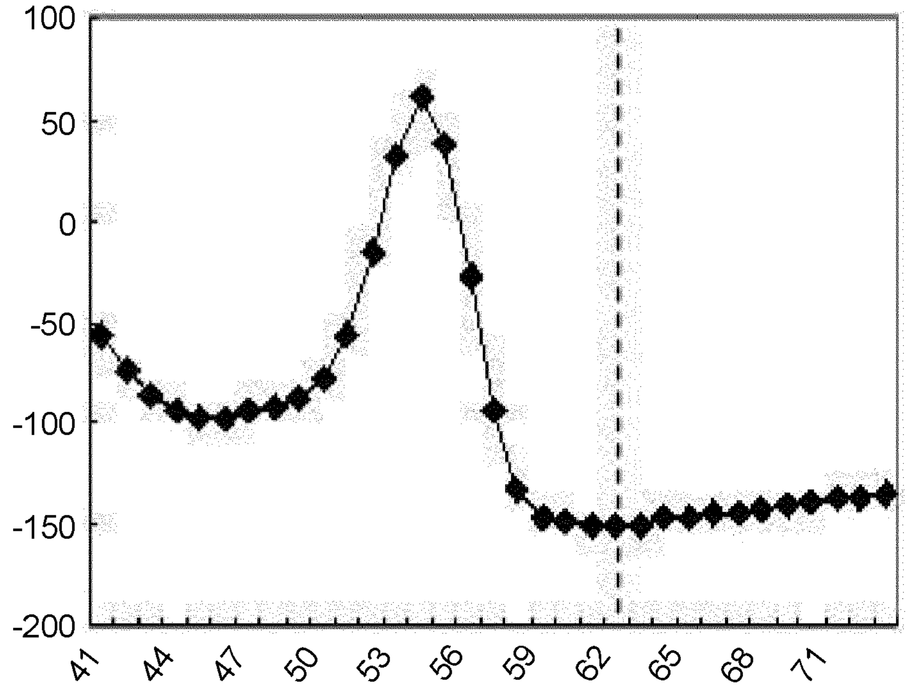
100
50
0
-50
-100
-150
-200
41
44
47
50
53
56
59
62
65
68
71

FIG. 4A

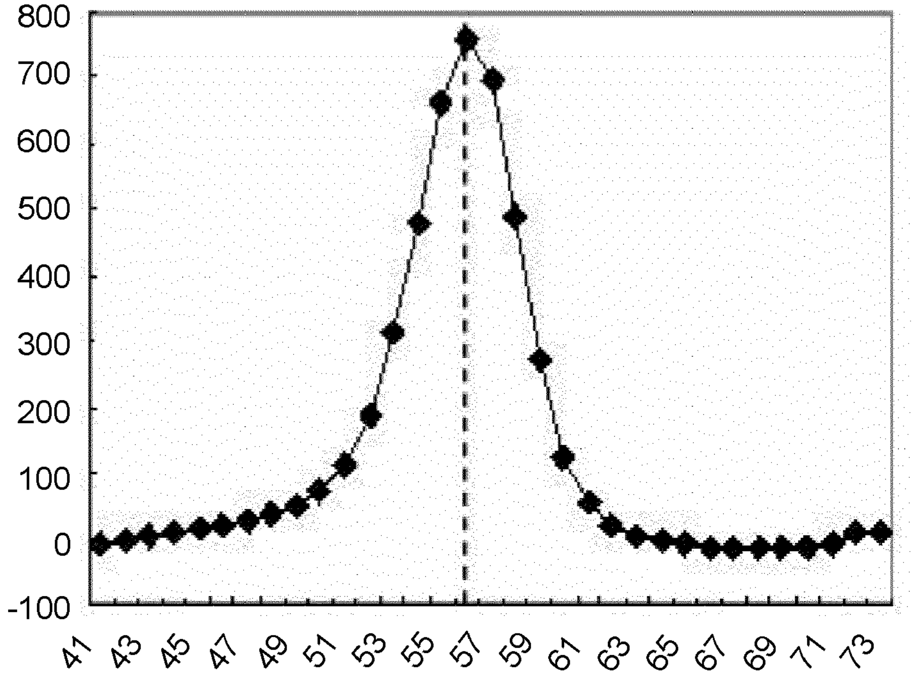
800
700
600
500
400
300
200
100
0
-100
41
43
45
47
49
51
53
55
57
59
61
63
65
67
69
71
73

FIG. 4B

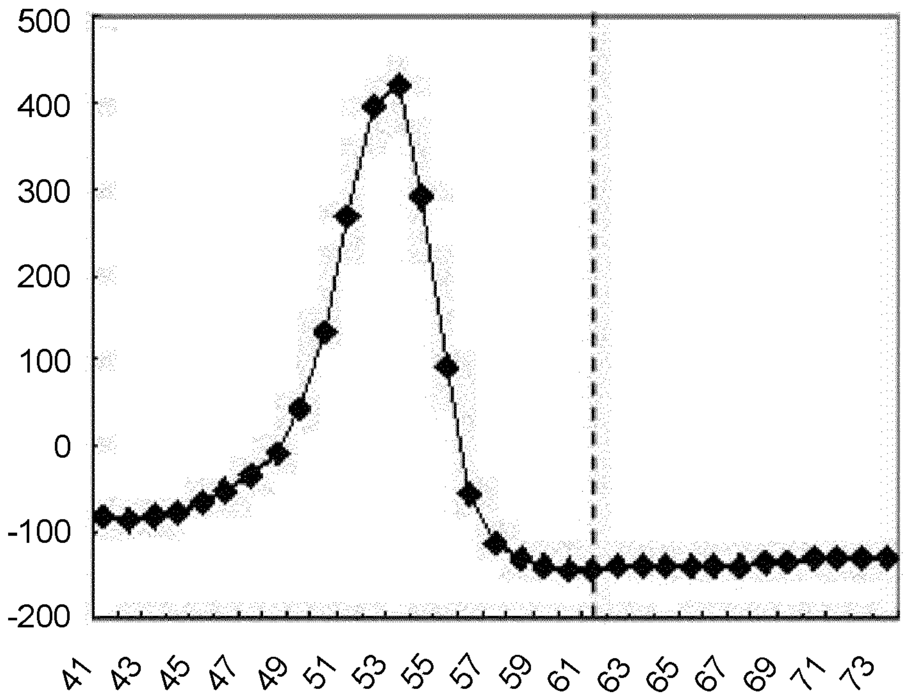
500
400
300
200
100
0
-100
-200
41
43
45
47
49
51
53
55
57
59
61
63
65
67
69
71
73

FIG. 5A

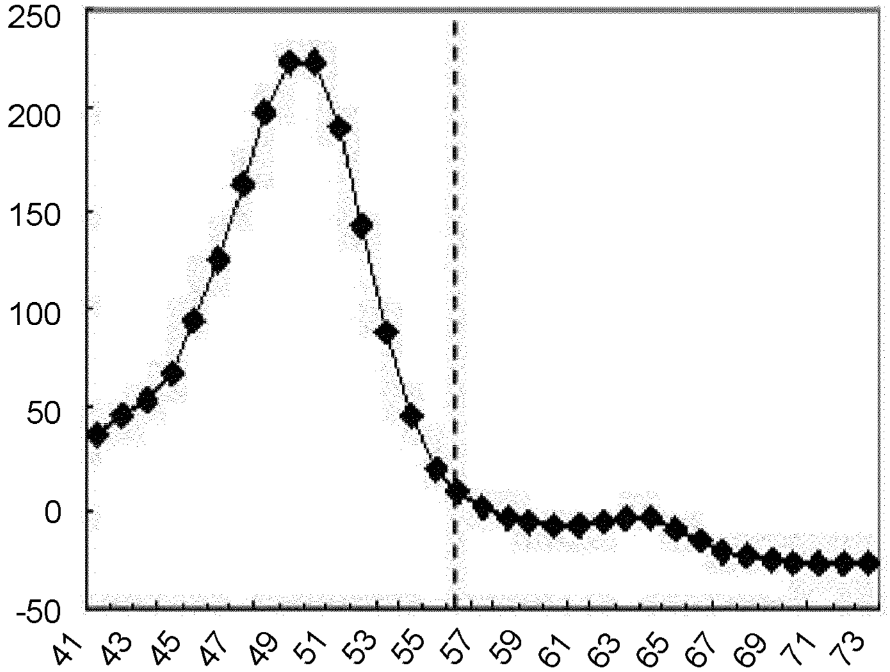
250
200
150
100
50
0
-50
41
43
45
47
49
51
53
55
57
59
61
63
65
67
69
71
73

FIG. 5B

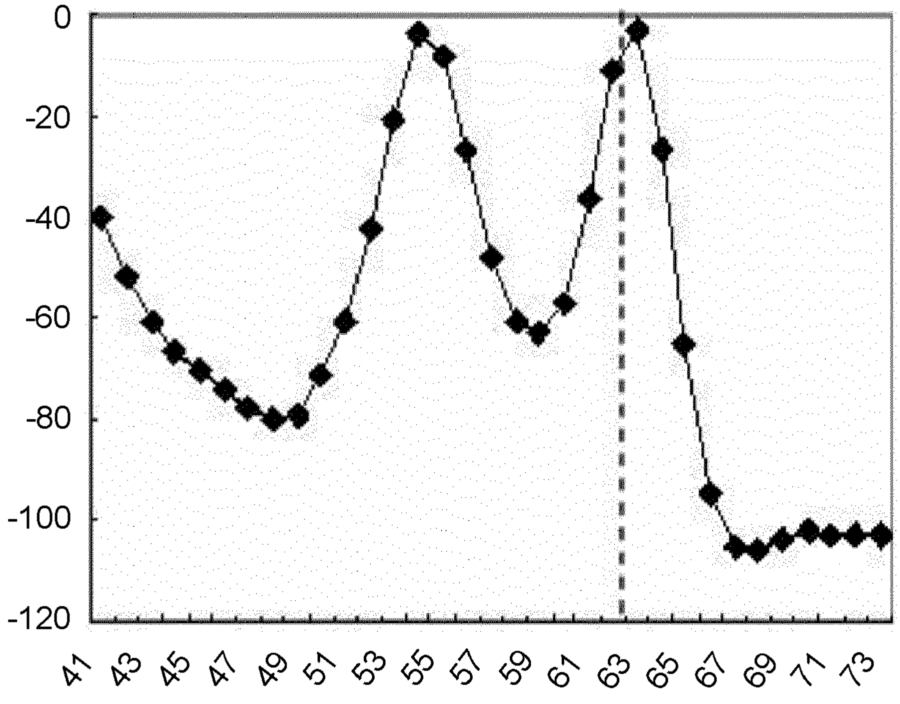
0
-20
-40
-60
-80
-100
-120
41
43
45
47
49
51
53
55
57
59
61
63
65
67
69
71
73

FIG. 6A

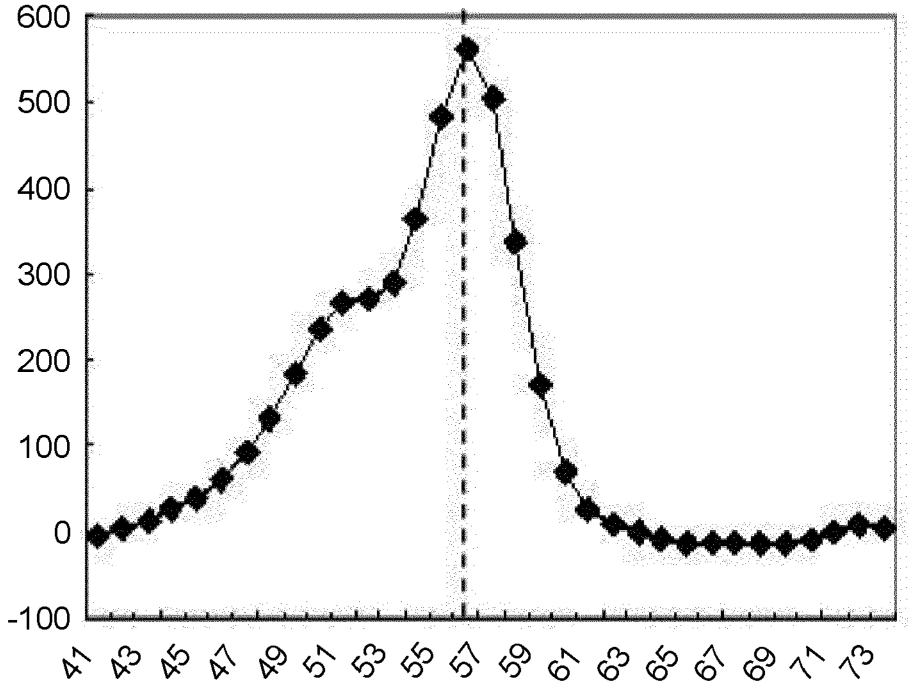
600
500
400
300
200
100
0
-100
41 43 45 47 49 51 53 55 57 59 61 63 65 67 69 71 73

FIG. 6B

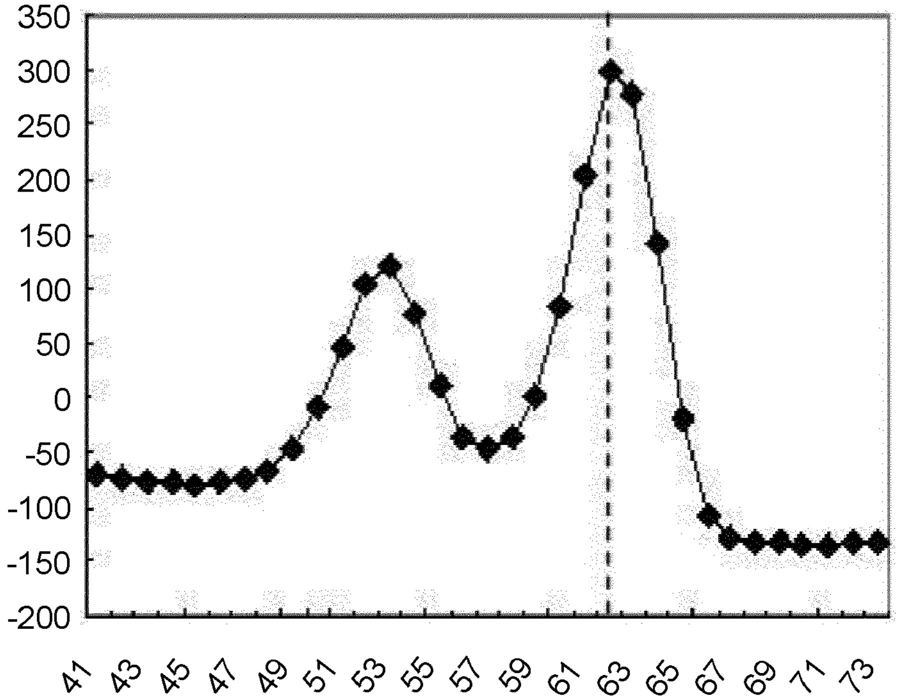
350
300
250
200
150
100
50
0
-50
-100
-150
-200
41 43 45 47 49 51 53 55 57 59 61 63 65 67 69 71 73

FIG. 7A

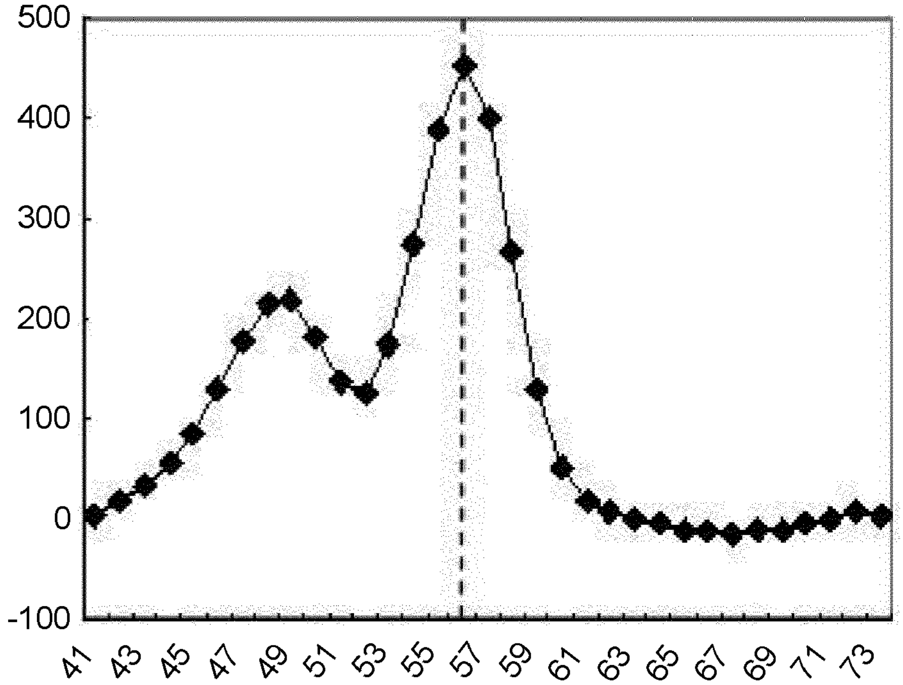
500
400
300
200
100
0
-100
41 43 45 47 49 51 53 55 57 59 61 63 65 67 69 71 73

FIG. 7B

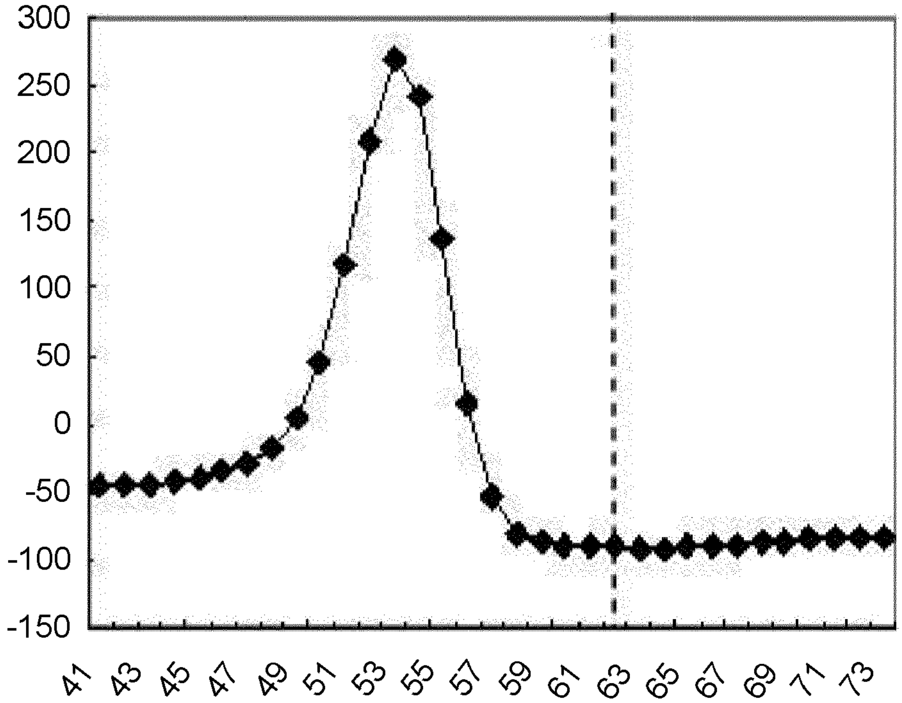
300
250
200
150
100
50
0
-50
-100
-150
41 43 45 47 49 51 53 55 57 59 61 63 65 67 69 71 73

FIG. 8

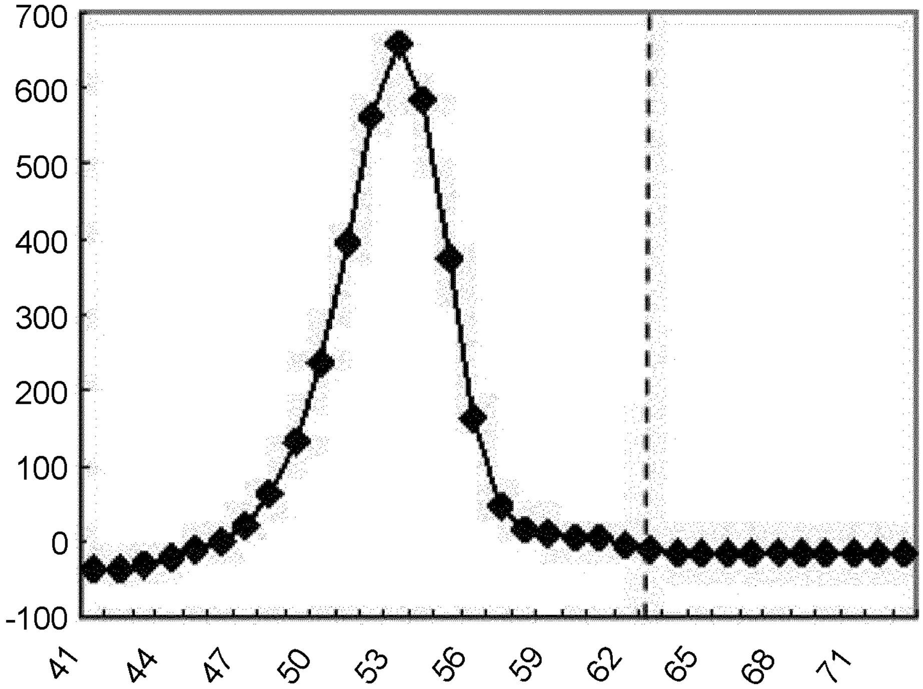
700
600
500
400
300
200
100
0
-100
41
44
47
50
53
56
59
62
65
68
71

FIG. 9

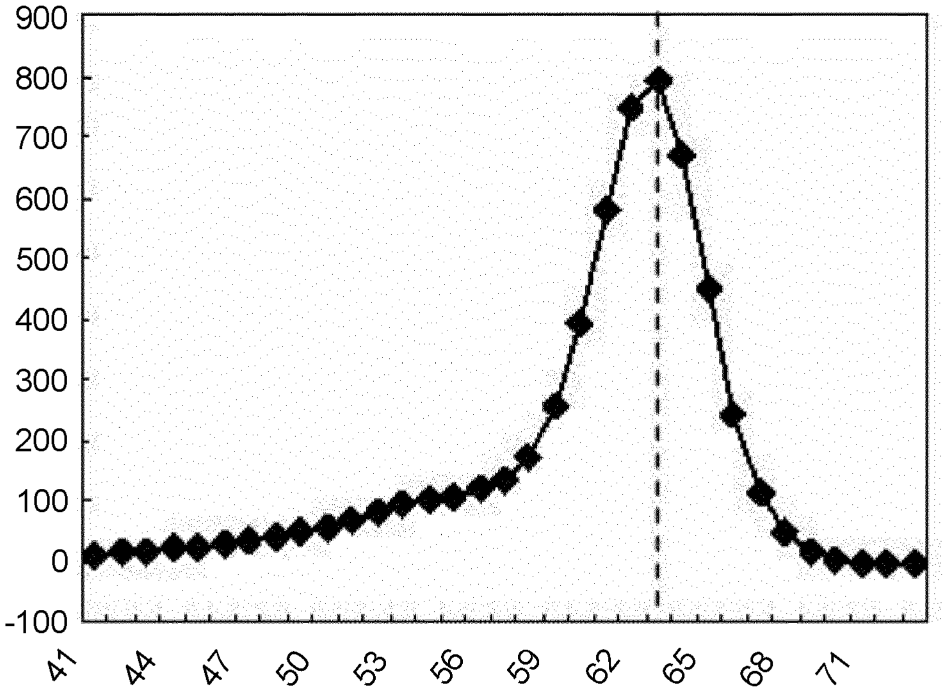
900
800
700
600
500
400
300
200
100
0
-100
41
44
47
50
53
56
59
62
65
68
71

FIG. 10

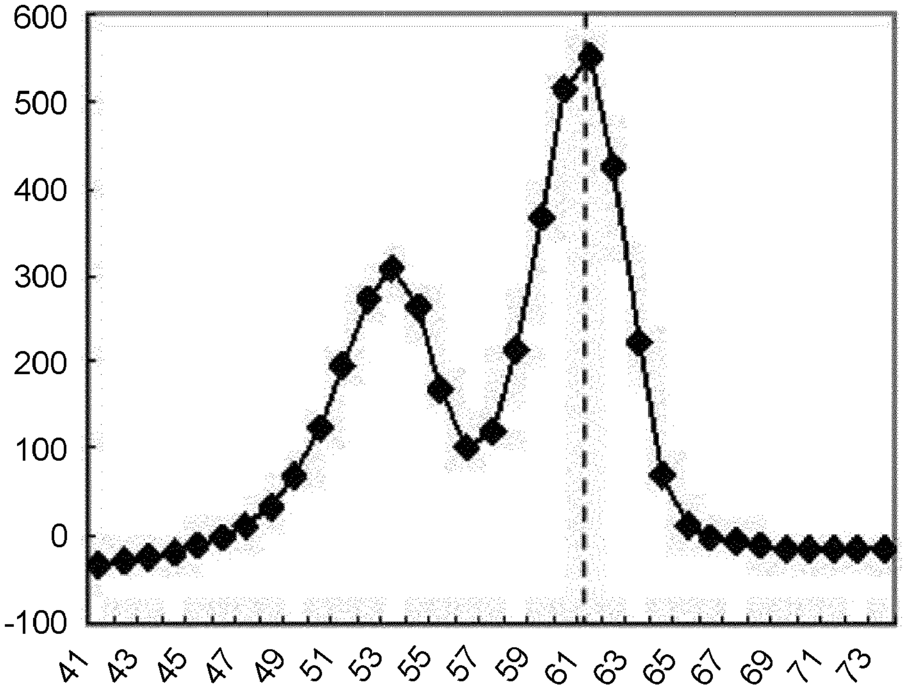
600
500
400
300
200
100
0
-100
41
43
45
47
49
51
53
55
57
59
61
63
65
67
69
71
73

Europäisches Patentamt
European Patent Office
Office européen des brevets

# EUROPEAN SEARCH REPORT

Application Number
EP 11 18 7128

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 01/42509 A1 (CHEO DAVID [US]; BRASCH MICHAEL A [US]; TEMPLE GARY F [US]; HARTLEY JA) 14 June 2001 (2001-06-14)<br>* page 213, line 6;<br>page 68, lines 13-19;<br>page 100, line 21 - page 101, line 4 *<br>----- | 1-7, 13-15<br><br><br>12 | INV. C12Q1/68 |
| X<br><br><br>Y | WO 2009/113580 A1 (JAPAN SCIENCE & TECH AGENCY [JP])<br>17 September 2009 (2009-09-17)<br>* Example 7; par. 120 *<br>----- | 1-11<br><br><br>12 | |
| A,P | EP 2 270 015 A1 (JAPAN SCIENCE & TECH AGENCY [JP]) 5 January 2011 (2011-01-05)<br>* Example 7; par. 118 *<br>----- | 1-12 | |
| Y | C. SIMON ET AL: "Intestinal expression of cytochrome P450 enzymes and ABC transporters and carbamazepine and phenytoin disposition",<br>ACTA NEUROLOGICA SCANDINAVICA,<br>vol. 115, no. 4, 1 April 2007 (2007-04-01), pages 232-242, XP55020065,<br>ISSN: 0001-6314, DOI: 10.1111/j.1600-0404.2006.00761.x<br>* Table 3 *<br>* the whole document *<br>----- | 12 | TECHNICAL FIELDS SEARCHED (IPC)<br>C12Q |

The present search report has been drawn up for all claims

| Place of search | Date of completion of the search | Examiner |
|---|---|---|
| Munich | 1 March 2012 | Leber, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 11 18 7128

This annex lists the patent family members relating to the patent documents cited in the above-mentioned European search report. The members are as contained in the European Patent Office EDP file on
The European Patent Office is in no way liable for these particulars which are merely given for the purpose of information.

01-03-2012

| Patent document cited in search report | Publication date | Patent family member(s) | Publication date |
|---|---|---|---|
| WO 0142509 A1 | 14-06-2001 | AT 391789 T | 15-04-2008 |
| | | AU 785483 B2 | 20-09-2007 |
| | | AU 2085101 A | 18-06-2001 |
| | | AU 2007221891 A1 | 01-11-2007 |
| | | CA 2392753 A1 | 14-06-2001 |
| | | CN 1468317 A | 14-01-2004 |
| | | DE 60038572 T2 | 30-04-2009 |
| | | DK 1250453 T3 | 11-08-2008 |
| | | EP 1250453 A1 | 23-10-2002 |
| | | EP 1978098 A2 | 08-10-2008 |
| | | EP 2210948 A2 | 28-07-2010 |
| | | HK 1062034 A1 | 15-10-2010 |
| | | JP 4729222 B2 | 20-07-2011 |
| | | JP 2004500061 A | 08-01-2004 |
| | | NZ 519217 A | 26-03-2004 |
| | | NZ 530816 A | 28-10-2005 |
| | | NZ 539430 A | 29-09-2006 |
| | | WO 0142509 A1 | 14-06-2001 |
| WO 2009113580 A1 | 17-09-2009 | CA 2722479 A1 | 17-09-2009 |
| | | CN 101970443 A | 09-02-2011 |
| | | EP 2270015 A1 | 05-01-2011 |
| | | US 2011033863 A1 | 10-02-2011 |
| | | WO 2009113580 A1 | 17-09-2009 |
| EP 2270015 A1 | 05-01-2011 | CA 2722479 A1 | 17-09-2009 |
| | | CN 101970443 A | 09-02-2011 |
| | | EP 2270015 A1 | 05-01-2011 |
| | | US 2011033863 A1 | 10-02-2011 |
| | | WO 2009113580 A1 | 17-09-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002119291 A
- JP 4454366 B
- JP 2005028733 A
- JP 2005287335 A


### Non-patent literature cited in the description


- *Proc. Natl. Acad. Sci. USA.,* 2000, vol. 97 (7), 3473-3478
- *Genet. Mol. Res.,* 2010, vol. 9 (1), 34-40
- *Br. J. Clin. Pharmcol.,* 2005, vol. 59 (3), 365-370
- *Clin. Chem.,* 2000, vol. 46 (5), 631-635

SEQUENCE LISTING

<110> Arkray, Inc.

<120> POLYMORPHISM DETECTION PROBE, POLYMORPHISM DETECTION METHOD, EVALUATION OF DRUG EFFICACY, AND POLYMORPHISM DETECTION KIT

<130> 42.13.110997

<150> JP2010-242836
<151> 2010-10-28

<150> JP2011-235652
<151> 2011-10-27

<160> 43

<170> PatentIn version 3.4

<210> 1
<211> 511
<212> DNA
<213> Homo sapiens

<220>
<221> misc_feature
<222> (256)..(256)
<223> n is c or t

<400> 1
ctcacagtaa cttggcagtt tcagtgtaag aaataatgat gttaattgtg ctacattcaa 60

agtgtgctgg tcctgaagtt gatctgtgaa ctcttgtttt cagctgcttg atggcaaaga 120

aataaagcga ctgaatgttc agtggctccg agcacacctg ggcatcgtgt cccaggagcc 180

catcctgttt gactgcagca ttgctgagaa cattgcctat ggagacaaca gccgggtggt 240

gtcacaggaa gagatngtga gggcagcaaa ggaggccaac atacatgcct tcatcgagtc 300

actgcctaat gtaagtctct cttcaaataa acagcctggg agcatgtggc agcctctctg 360

gcctatagtt tgatttataa ggggctggtt tcccagaagt gaagagaaat tagcaaccaa 420

atcacaccct tacctgtata caagcatctg gccacacttc ctgtttgggt tagttgttac 480

ctttacctga tcacctgacc ctccttgtga g 511

<210> 2
<211> 600
<212> DNA
<213> Homo sapiens

<220>
<221> misc_feature
<222> (300)..(300)
<223> n is g, t or a

<400> 2
taaaataatg aatatagtct catgaaggtg agttttcaga aaatagaagc atgagttgtg 60

aagataatat ttttaaaatt tctctaattt gttttgtttt gcaggctrta ggttccaggc 120

ttgctgtaat tacccagaat atagcaaatc ttgggacagg aataattata tccttcatct 180

atggttggca actaacactg ttactcttag caattgtacc catcattgca atagcaggag 240

ttgttgaaat gaaaatgttg tctggacaag cactgaaaga taagaaagaa ctagaaggtn 300

ctgggaaggt gagtcaaact aaatatgatt gattaattaa gtagagtaaa gtattcyaat 360

cagtgttatt ttgtyactcc ctactgctta ctatgctcta agaatgtgtt tataaccatt 420

cctcaaagca atctttttca tgcttattca gtaaattaga aacttacaga aagtagcaaa 480

gccagttctt ggactcaaaa actgataatt aactttaaca gactttttca gttttcaggc 540

cattgtcttc acactgttct tccttcctcc ccactttcct ccttccctta gttatkttct 600

<210> 3
<211> 23
<212> DNA
<213> Artificial

<220>
<223> primer

<400> 3
aaatgttgtc tggacaagca ctg 23

<210> 4
<211> 30
<212> DNA
<213> Artificial

<220>
<223> primer

<400> 4
aattaatcaa tcatatttag tttgactcac 30

<210> 5
<211> 20
<212> DNA
<213> Artificial

<220>
<223> primer

<400> 5
actgcagcat tgctgagaac 20

<210> 6
<211> 21
<212> DNA
<213> Artificial

<220>
<223> primer

<400> 6
cagagaggct gccacatgct c 21

<210> 7
<211> 19
<212> DNA
<213> Artificial

<220>
<223> probe

<400> 7
ctgccctcac aatctcttc 19

<210> 8
<211> 21
<212> DNA
<213> Artificial

<220>
<223> probe

<400> 8
cttcccagca ccttctagtt c 21

<210> 9
<211> 18
<212> DNA
<213> Artificial

<220>
<223> probe

<400> 9
cccagaacct tctagttc 18

<210> 10
<211> 18
<212> DNA
<213> Artificial

<220>
<223> probe

<400> 10
ctagaaggtt ctgggaag 18

<210> 11
<211> 13
<212> DNA
<213> Artificial

<220>
<223> probe

<400> 11
caccttctag ttc 13

<210> 12
<211> 24
<212> DNA
<213> Artificial

<220>
<223> probe

<400> 12
caccttccca gcaccttcta gttc 24

<210> 13
<211> 25
<212> DNA
<213> Artificial

<220>
<223> probe

<400> 13
tcaccttccc agcaccttct agttc 25

<210> 14
<211> 26
<212> DNA
<213> Artificial

<220>
<223> probe

<400> 14
ctcaccttcc cagcaccttc tagttc 26

<210> 15
<211> 31
<212> DNA
<213> Artificial

<220>
<223> probe

<400> 15
tttgactcac cttcccagca ccttctagtt c 31

<210> 16
<211> 36
<212> DNA
<213> Artificial

<220>
<223> probe

<400> 16
tttagtttga ctcaccttcc cagcaccttc tagttc 36

<210> 17
<211> 41
<212> DNA
<213> Artificial

<220>
<223> probe

<400> 17

tcatatttag tttgactcac cttcccagca ccttctagtt c 41

<210> 18
<211> 46
<212> DNA
<213> Artificial

<220>
<223> probe

<400> 18
atcaatcata tttagtttga ctcaccttcc cagcaccttc tagttc 46

<210> 19
<211> 51
<212> DNA
<213> Artificial

<220>
<223> probe

<400> 19
aattaatcaa tcatatttag tttgactcac cttcccagca ccttctagtt c 51

<210> 20
<211> 56
<212> DNA
<213> Artificial

<220>
<223> probe

<400> 20
tacttaatta atcaatcata tttagtttga ctcaccttcc cagcaccttc tagttc 56

<210> 21
<211> 61
<212> DNA
<213> Artificial

<220>
<223> probe

<400> 21
tactctactt aattaatcaa tcatatttag tttgactcac cttcccagca ccttctagtt 60

c 61

<210> 22
<211> 66
<212> DNA
<213> Artificial

<220>
<223> probe

<400> 22
tactttactc tacttaatta atcaatcata tttagtttga ctcaccttcc cagcaccttc 60

tagttc 66

<210> 23
<211> 67
<212> DNA
<213> Artificial

<220>
<223> probe

<400> 23
atactttact ctacttaatt aatcaatcat atttagtttg actcaccttc ccagcacctt 60

ctagttc 67

<210> 24
<211> 68
<212> DNA
<213> Artificial

<220>
<223> probe

<400> 24
aatactttac tctacttaat taatcaatca tatttagttt gactcacctt cccagcacct 60

tctagttc 68

<210> 25
<211> 10
<212> DNA
<213> Artificial

<220>
<223> probe

<400> 25
cccagaaaaa 10

<210> 26
<211> 23
<212> DNA
<213> Artificial

<220>
<223> probe

<400> 26
cccagaacct tctagttctt tct 23

<210> 27
<211> 28
<212> DNA
<213> Artificial

<220>
<223> probe

<400> 27
cccagaacct tctagttctt tcttatct 28

<210> 28
<211> 29
<212> DNA
<213> Artificial

<220>
<223> probe

<400> 28
cccagaacct tctagttctt tcttatctt 29

<210> 29
<211> 33
<212> DNA
<213> Artificial

<220>
<223> probe

<400> 29
cccagaacct tctagttctt tcttatcttt cag 33

<210> 30
<211> 38
<212> DNA
<213> Artificial

<220>
<223> probe

<400> 30
cccagaacct tctagttctt tcttatcttt cagtgctt 38

<210> 31
<211> 43
<212> DNA
<213> Artificial

<220>
<223> probe

<400> 31
cccagaacct tctagttctt tcttatcttt cagtgcttgt cca 43

<210> 32
<211> 48
<212> DNA
<213> Artificial

<220>
<223> probe

<400> 32
cccagaacct tctagttctt tcttatcttt cagtgcttgt ccagacaa 48

<210> 33
<211> 53
<212> DNA
<213> Artificial

<220>
<223> probe

<400> 33
cccagaacct tctagttctt tcttatcttt cagtgcttgt ccagacaaca ttt 53

<210> 34
<211> 58
<212> DNA
<213> Artificial

<220>
<223> probe

<400> 34
cccagaacct tctagttctt tcttatcttt cagtgcttgt ccagacaaca ttttcatt 58

<210> 35
<211> 63
<212> DNA
<213> Artificial

<220>
<223> probe

<400> 35
cccagaacct tctagttctt tcttatcttt cagtgcttgt ccagacaaca ttttcatttc 60

aac 63

<210> 36
<211> 68
<212> DNA
<213> Artificial

<220>
<223> probe

<400> 36
cccagaacct tctagttctt tcttatcttt cagtgcttgt ccagacaaca ttttcatttc 60

aacaactc 68

<210> 37
<211> 73
<212> DNA
<213> Artificial

<220>
<223> probe

<400> 37
cccagaacct tctagttctt tcttatcttt cagtgcttgt ccagacaaca ttttcatttc 60

aacaactcct gct 73

<210> 38
<211> 78
<212> DNA

<213> Artificial

<220>
<223> probe

<400> 38
cccagaacct tctagttctt tcttatcttt cagtgcttgt ccagacaaca ttttcatttc 60

aacaactcct gctattgc 78

<210> 39
<211> 83
<212> DNA
<213> Artificial

<220>
<223> probe

<400> 39
cccagaacct tctagttctt tcttatcttt cagtgcttgt ccagacaaca ttttcatttc 60

aacaactcct gctattgcaa tga 83

<210> 40
<211> 88
<212> DNA
<213> Artificial

<220>
<223> probe

<400> 40
cccagaacct tctagttctt tcttatcttt cagtgcttgt ccagacaaca ttttcatttc 60

aacaactcct gctattgcaa tgatgggt 88

<210> 41
<211> 93
<212> DNA
<213> Artificial

<220>
<223> probe

<400> 41
cccagaacct tctagttctt tcttatcttt cagtgcttgt ccagacaaca ttttcatttc 60

aacaactcct gctattgcaa tgatgggtac aat 93

<210> 42
<211> 69
<212> DNA
<213> Artificial

<220>
<223> probe

<400> 42
gaatacttta ctctacttaa ttaatcaatc atatttagtt tgactcacct tcccagcacc 60

ttctagttc 69

```
<210>  43
<211>  94
<212>  DNA
<213>  Artificial

<220>
<223>  probe

<400>  43
cccagaacct tctagttctt tcttatcttt cagtgcttgt ccagacaaca ttttcatttc     60

aacaactcct gctattgcaa tgatgggtac aatt                                 94
```